(19) Europäisches Patentamt
European Patent Office
Office européen des brevets

(11) EP 4 065 176 B1

(12) EUROPEAN PATENT SPECIFICATION

(45) Date of publication and mention of the grant of the patent:
**11.10.2023 Bulletin 2023/41**

(21) Application number: **21810930.4**

(22) Date of filing: **05.11.2021**

(51) International Patent Classification (IPC):
***A61K 51/08*** *(2006.01)* ***A61K 103/30*** *(2006.01)*
***A61K 31/196*** *(2006.01)* ***A61P 35/00*** *(2006.01)*

(52) Cooperative Patent Classification (CPC):
**A61P 35/00; A61K 31/196; A61K 51/083; A61K 51/088**

(86) International application number:
**PCT/EP2021/080822**

(87) International publication number:
**WO 2022/096673 (12.05.2022 Gazette 2022/19)**

# (54) COMBINATION OF PARA-AMINOHIPPURIC ACID (PAH) AND RADIOLABELED COMPLEXES FOR TREATING CANCER

KOMBINATION VON PARA-AMINOHIPPURSÄURE (PAH) UND RADIOAKTIV MARKIERTEN KOMPLEXEN ZUR BEHANDLUNG VON KREBS

COMBINAISON D'ACIDE PARA-AMINOHIPPURIQUE (PAH) ET DE COMPLEXES RADIOMARQUÉS POUR TRAITER LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.11.2020 PCT/EP2020/081143**

(43) Date of publication of application:
**05.10.2022 Bulletin 2022/40**

(73) Proprietor: **ITM Isotope Technologies Munich SE**
**85748 Garching bei München (DE)**

(72) Inventors:
- **MECKEL, Marian**
  **85748 Garching/Munich (DE)**
- **OSL, Theresa**
  **85748 Garching/Munich (DE)**
- **ZHERNOSEKOV, Konstantin**
  **85748 Garching/Munich (DE)**

(74) Representative: **Graf von Stosch Patentanwaltsgesellschaft mbH**
**Prinzregentenstraße 22**
**80538 München (DE)**

(56) References cited:
**WO-A1-2020/225447 WO-A2-2008/124660**

- **BAUM RICHARD P. ET AL: "[177Lu-DOTA]0-D-Phe1-Tyr3-Octreotide (177Lu-DOTATOC) For Peptide Receptor Radiotherapy in Patients with Advanced Neuroendocrine Tumours: A Phase-II Study", THERANOSTICS, vol. 6, no. 4, 13 February 2016 (2016-02-13), pages 501-510, XP055822256, AU ISSN: 1838-7640, DOI: 10.7150/thno.13702**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 065 176 B1

## Description

The present invention relates to the field of radiopharmaceuticals for the treatment of cancer, in particular to combination therapies with radiopharmaceuticals for the treatment of cancer.

Radiopharmaceuticals are drugs, which contain radioactive isotopes (radionuclides). Radiopharmaceuticals can be used to treat various conditions, including cancers, blood disorders and hyperthyroidism. In radiopharmaceutical therapy of cancer, a molecule labeled with a radionuclide is used to deliver a toxic level of radiation to disease sites. Thereby, the molecule is used to "target" the disease site, e.g. specific cancer cells, for instance by binding specifically to the targeted cancer cells or by accumulating by a wide variety of physiological mechanisms characteristic of neoplasia. Accordingly, the radionuclide complex combines the specificity of cancer targeting with the known antitumor effects of ionizing radiation. With radiopharmaceuticals not only the primary tumor site, but also its metastases can be targeted. The choice of the molecule that carries the radiation to the tumor is usually determined by its selectivity and affinity to the tumor's target structures, such as antigens or receptors. Even if a target structure is not selective for a certain kind of cancer, overexpressed target structures are of interest, because they allow the delivery of the radionuclide complex in high concentration to those (overexpressing) target cells while leaving other cells (with no or minor expression only) essentially unaffected. Radionuclides are usually linked to the targeting molecule through chelating agents. Thereby, strong complexes with the metal ion of the radionuclide can be formed.

Radiopharmaceuticals have shown efficacy with minimal toxicity compared with almost all other systemic cancer treatment options. In the recent years, the FDA approved various novel radiopharmaceutical therapies (RPTs): the $\alpha$-emitter Radium-223 ($^{223}$Ra) for bone metastases of castrate-resistant prostate cancer, [$^{177}$Lu]Lu-DOTATATE (Lutathera®) for the treatment of somatostatin receptor (SSTR) positive gastroenteropancreatic neuroendocrine tumors, and [$^{111}$I]I-mIBG for malignant pheochromocytoma and paraganglioma. Other approved radiopharmaceuticals include $^{153}$Sm-EDTMP (Quadramet®, which uses ethylenediaminetetramethylenephosphonic acid (EDTMP) as chelator, binding samarium-153 through six ligands), strontium-89-chloride for palliation of bone metastases, $^{90}$Y- loaded microspheres (which may be glass based (TheraSphere™) or resin based (SIR-Sphere®)), and yttrium-90 ibritumomab tiuxetan (Zevalin®) for treating indolent B-cell lymphoma and related cancers.

Baum et al., Theranostics 2016, vol. 6, issue 4, p. 501, discloses the use of the radiolabeled somatostatin analog $^{177}$Lu-DOTATOC in peptide receptor radiotherapy of neuroendocrine tumors.

Despite the efficacy of radiopharmaceuticals in the treatment of cancer, the development of biological resistance to these agents, which may be due to outgrowth of cancer cells with low or no target expression, must be considered. Moreover, for cancer therapeutics generally an increase in efficacy, in particular with regard to reducing or delaying tumor growth and extending survival times is desired.

Para-aminohippuric acid (PAH) is a derivative of hippuric acid, that is not naturally found in humans. It is known as diagnostic agent in the measurement of renal plasma flow, in particular to measure effective renal plasma flow (ERPF) and excretory capacity. PAH was also described to reduce nephrotoxic effects of cisplatin (Natochin et al., 1989, Comp. Biochem. Physiol Vol. 94C, No.1 pp. 115-120). Unpublished patent application PCT/EP2020/062950 discloses that PAH reduces nephrotoxic effects of radiolabeled compounds.

In view of the above, it is the object of the present invention to overcome the drawbacks outlined above and to provide a novel combination of (i) a radionuclide complex and (ii) para-aminohippuric acid (PAH) for the treatment of cancer. In particular, it is the object of the present invention to provide a novel combination therapy for radiopharmaceuticals, which increases the efficacy of radiopharmaceuticals in cancer treatment.

This object is achieved by means of the subject-matter set out below and in the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In

the context of the present invention, the term "comprise" encompasses the term "consist of". The term "comprising" thus encompasses "including" as well as "consisting" e.g., a composition "comprising" X may consist exclusively of X or may include something additional *e.g.*, X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g.,* a composition which is "substantially free" from Y may be completely free from Y. Where necessary, the word "substantially" may be omitted from the definition of the invention.

The term "about" in relation to a numerical value x means x $\pm$ 20%, preferably x $\pm$ 10%, more preferably x $\pm$ 5%, even more preferably x $\pm$ 2% and still more preferably x $\pm$ 1%.

***Combination of PAH and a radiolabeled complex for treating cancer***

In a first aspect the present invention provides a combination comprising:

(a) a radiolabeled complex comprising (i) a radionuclide and (ii) a targeting molecule linked to a chelating agent, wherein the targeting molecule is selected from peptides, peptidomimetics, antibody fragments, antibody mimetics and folate; and

(b) para-aminohippuric acid (PAH); or a salt thereof; for use in the treatment of cancer, wherein the combined administration of (a) and (b) increases the anti-tumor efficacy of (a).

The present inventors have surprisingly found that combined administration of a radiolabeled complex and PAH even further increases the anti-tumor effects of the radiolabeled complex. In particular, tumor growth was even further reduced/delayed as compared to radiopharmaceutical monotherapy. Likewise, in combination with PAH even further increased survival times were observed as compared to radiopharmaceutical monotherapy. Accordingly, combined administration of radiopharmaceuticals with PAH surprisingly even further improved the anti-tumor efficacy of radiopharmaceuticals. As PAH was previously known as diagnostic agent in the measurement of renal plasma flow and as nephroprotective agent to reduce nephrotoxic side effects of certain medicaments, its efficacy to improve the anti-tumor action of radiopharmaceuticals, as found by the present inventors, was completely unexpected.

As used herein, the term "combination" refers to any kind of combination of its components, in particular, to any kind of combination of (a) the radiolabeled complex and (b) PAH (or a salt or carboxylic acid derivate thereof) and, optionally, any further components. In particular, the components of a combination are provided and/or administered together (i.e., in a combined manner). In some embodiments, the combination may be a kit (e.g., comprising the components in an (at least partially) separated manner). In other embodiments, the combination may be a composition (e.g., the components may be comprised in a single composition).

Radiolabeled complex

Radiopharmaceuticals may comprise nonmetallic (organic) radionuclides ($^{18}$F, $^{11}$C, $^{13}$N, $^{15}$O, $^{124}$I, etc.) or radiometals (e.g. $^{90}$Y, $^{99m}$Tc, $^{111}$In, $^{131}$1, $^{67}$Ga, $^{68}$Ga, $^{64}$Cu, $^{161}$Tb, $^{225}$Ac, $^{44}$Sc, $^{47}$Sc, $^{67}$Cu, $^{89}$Zr, $^{177}$Lu, etc.). Although some radiometals can target a particular tissue as a metal salt or as a metal complex, it is mostly required to conjugate the radionuclide/radiometal with a targeting biomolecule ("targeting molecule") so that the radionuclide is delivered to the target site, e.g. the tumor tissue, in a targeted manner. The targeting molecules can, e.g., be peptides, monoclonal antibodies (mAbs) or mAbs fragments. They serve as the vehicle ("carrier", "targeting molecule") to carry the radionuclide to the target tissue. The most elegant approach to establish a stable conjugation of a radionuclide and a targeting biomolecule (carrier) is to use a (bifunctional) chelator or chelating agent, which typically binds or coordinates the radionuclide tightly and, at the same time, presents functional moieties for its conjugation with the biomolecule. Accordingly, the radiolabeled complex, as used in the present invention, preferably comprises (i) a radionuclide and (ii) a targeting molecule linked to a chelating agent.

Various radiolabeled complexes comprising (i) the radionuclide and (ii) the targeting molecule linked to a chelating agent are known in the art. Particularly preferred examples of radiolabeled complexes are described in WO 2018/215627 A1.

Further examples for commercially available radiolabeled complexes, which may be combined with PAH to

treat cancer, as described herein, include [$^{177}$Lu]Lu-DOTATATE (Lutathera®), [$^{131}$I]I-mIBG, $^{153}$Sm-EDTMP (Quadramet®), $^{89}$Sr chloride, $^{90}$Y-loaded microspheres (TheraSphere™ or SIR-Sphere®), and yttrium-90 ibritumomab tiuxetan (Zevalin®).

In radioligand therapies (also known as "radiopharmaceutical therapy" and "peptide-receptor radionuclide therapy") radiopharmaceuticals are labeled by a radioligand, which usually specifically binds to a (tumor) cell target, e.g. a tumor cell surface protein or marker. After binding of the compound to the tumor target, for example to a receptor, the radionuclide releases energetic alpha or beta particle radiation to precisely target cells at the targeted site.

*Radionuclide*

Various radionuclides (radioisotopes) are known to be useful in the field of radionuclide therapy. In particular, the term "radionuclide" (or "radioisotope") refers to isotopes of natural or artificial origin with an unstable neutron to proton ratio that disintegrates with the emission of corpuscular (i.e. protons (alpha-radiation) or electrons (beta-radiation) or electromagnetic radiation (gamma-radiation). In other words, radionuclides undergo radioactive decay. Said radionuclide which is preferably useful for the treatment of cancer. Non-limiting examples of suitable radionuclides include $^{18}$F, $^{131}$I, $^{94}$Tc, $^{99m}$Tc, $^{90}$In, $^{111}$In, $^{67}$Ga, $^{68}$Ga, $^{86}$Y, $^{90}$Y, $^{177}$Lu, $^{151}$Tb, $^{186}$Re, $^{188}$Re, $^{64}$Cu, $^{67}$Cu, $^{55}$Co, $^{57}$Co, $^{43}$Sc, $^{44}$Sc, $^{47}$Sc, $^{211}$At, $^{225}$Ac, $^{213}$Bi, $^{212}$Bi, $^{212}$Pb, $^{223}$Ra, $^{227}$Th, $^{153}$Sm, $^{166}$Ho, $^{152}$Gd, $^{153}$Gd, $^{157}$Gd, and $^{166}$Dy. Accordingly, the radionuclide may be any one of the before-mentioned examples.

For the treatment of cancer, radionuclides are preferred that emit ionizing radiation with short penetration into the tissue, such as $\alpha$ (alpha) or $\beta$ (beta) emitters, which release their energy in the proximity of their targets.

$\alpha$-emitters (a-particles) can travel 50-100 $\mu$m in tissue (only a few cell diameters), depending on their emission energy, $\alpha$-emitters are positively charged helium nuclei (two protons and two neutrons) that are emitted from the nucleus of a radioactive atom. $\alpha$-emitters are typically much larger than electrons (orders of magnitude) and exhibit high linear energy transfer. They can cause substantially more damage along their path than that caused by electrons, leading to greater biological effectiveness than either conventional external beam x-ray radiation or beta emitters. Preferred $\alpha$-particle emitters include, but are not limited to, $^{211}$At (astatine-211), $^{212}$Bi (bismuth-212), $^{212}$Pb (lead-212), $^{213}$Bi (bismuth-213), $^{225}$Ac (actinium-225), $^{223}$Ra (radium-223) and $^{227}$Th (thorium-227).

$\beta$-emitters ($\beta$-particles) are most frequently used in radioligand therapy of cancer. $\beta$-emitters are electrons emitted from the nucleus. They typically have a range in tissue of the order of about 1-5 mm. Accordingly, in the context of the present invention, radionuclides, which are $\beta$-emitters are preferred. $\beta$-emitters $^{153}$Sm (samarium-153), $^{177}$Lu (lutetium-177), $^{90}$Y (yttrium-90) and $^{131}$I (iodine-131) are commonly used over the last 40 years. For example, $^{131}$I (iodine-131) may be used to treat thyroid cancer. Preferred $\beta$-particle emitters include, but are not limited to, $^{90}$Y (yttrium-90), $^{131}$I (iodine-131), $^{153}$Sm (samarium-153), $^{177}$Lu (lutetium-177), and $^{89}$Sr (strontium-89), which are approved by the FDA for human use in targeted radiotherapeutics.

The choice of suitable radionuclides may depend *inter alia* on the chemical structure and chelating capability of the chelating agent, and the intended application of the resulting (complexed) conjugate (e.g., type and/or stage of cancer to be treated). For instance, the beta-emitters such as $^{90}$Y, $^{131}$1, $^{161}$Tb and $^{177}$Lu may be used for systemic radionuclide therapy. For example, DOTA, DOTAGA or DOTAM as chelating agent, may advantageously enable the use of $^{68}$Ga, $^{43,44,47}$Sc, $^{177}$Lu, $^{161}$Tb, $^{225}$Ac, $^{213}$Bi, $^{212}$Bi, or $^{212}$Pb as radionuclides.

Preferably, the radionuclide may be $^{131}$I or $^{90}$Y. Even more preferably, the radionuclide is $^{177}$Lu (Lutetium-177). Lutetium-177 emits photons in the 100-200-keV optimal imaging range and has a $\beta$-particle energy appropriate for therapy. Therefore, $^{177}$Lu is useful as theranostic, i.e. the same molecule can be used to assess tumor uptake and the (diagnostic) extent of cancer, but also as cancer treatment. Moreover, $^{177}$Lu exhibits a half-life, which is compatible with the pharmacokinetics of both antibodies and peptides. $^{177}$Lu is widely available and has a relatively straightforward conjugation chemistry.

*Chelating agent*

In the radiolabeled complex, the radionuclide metal ion is usually forming a non-covalent bond with functional groups of the chelating agent, e.g. amines or carboxylic acids. Typically, the chelating agent has at least two such complexing functional groups to be able to form a chelate complex.

As used herein, the term "chelating agent" (also referred to as "chelator") refers to polydentate (multiple bonded) ligands capable of forming two or more separate coordinate bonds with ("coordinating") a central (metal) ion, in particular the radionuclide metal ion. Specifically, such molecules or molecules sharing one electron pair may also be referred to as "Lewis bases". The central (metal) ion is usually coordinated by two or more electron pairs to the chelating agent. The terms, "bidentate chelating agent", "tridentate chelating agent", and "tetradentate chelating agent" are known in the art and refer to chelating agents having two, three, and four electron pairs, respectively, which are readily available for simultaneous donation to a metal ion coordinated by the chelating agent. Usually, the electron pairs of a chelating agent

forms coordinate bonds with a single central (metal) ion; however, in certain examples, a chelating agent may form coordinate bonds with more than one metal ion, with a variety of binding modes being possible.

The terms "coordinating" and "coordination" refer to an interaction in which one multi-electron pair donor coordinatively bonds (is "coordinated") to, i.e. shares two or more unshared pairs of electrons with, one central (metal) ion.

The chelator or chelating agent is preferably a macrocyclic bifunctional chelator having a metal chelating group at one end and a reactive functional group at the other end, which is capable to bind to other moieties, e.g. peptides. Preferably, the chelator may be selected such that the chelator forms a square bi-pyramidal complex for complexing the radionuclide. In another embodiment, the chelator does not from a planar or a square planar complex.

The chelating agent may be selected based on its ability to coordinate the desired central (metal) ion, usually the radionuclide as described herein. Preferably, the chelating agent is selected from 1,4,7,10-tetraazacyclododecane-1,4,7,10-tetraacetic acid (DOTA), N,N"-bis[2-hydroxy-5-(carboxyethyl)-benzyl]ethylenediamine-N,N"-diacetic acid (HBED-CC), 1,4,7-triazacyclononane-1 ,4,7-triacetic acid (NOTA), 2-(4,7-bis(carboxymethyl)-1,4,7-triazonan-1- yl)pentanedioic acid (NODAGA), 2-(4,7,10-tris(carboxymethyl)-1,4,7,10-tetraazacyclododecan-1-yl)-pentanedioic acid (DOTAGA), 1,4,7-triazacyclononane phosphinic acid (TRAP), 1,4,7-triazacydononane-1-[methyl(2-carboxyethyl)-phosphinic acid]-4,7-bis[methyl(2-hydroxymethyl)phosphinic acid] (NOPO), 3,6,9, 15-tetraazabicyclo[9,3,1]pentadeca-1(15),11,13-triene-3,6,9-triacetic acid (PCTA), N'-{5-[Acetyl(hydroxy)amino]pentyl}-N-[5-({4-[(5-aminopentyl)(hydroxy)amino]-4-oxobutanoyl}amino)pentyl]-N-hydroxysuccinamide (DFO), ethylenediaminetetraacetic acid (EDTA), nitrilotriacetic acid (NTA), 1,4,7,10-Tetraazacyclododecane-1,4,7-triacetic acid (DO3A), and Diethylen-etriaminepentaacetic acid (DTPA).

Accordingly, the chelating agent may be characterized by one of the following formulas (1a)-(1jj):

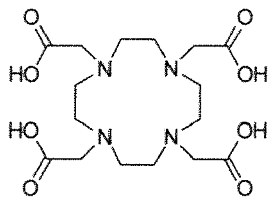

DOTA (1a)

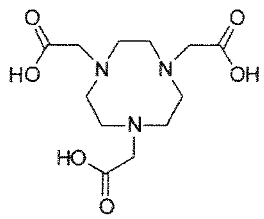

NOTA (1b)

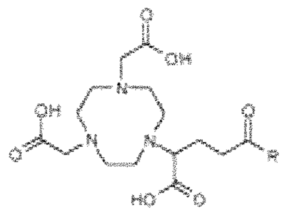

NODAGA (1c)

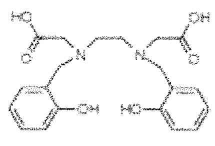

HBED (1d)

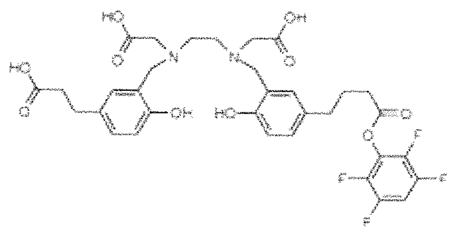

HBED-CC TFP (1e)

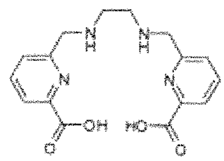

$H_2$DEPDPA (1f)

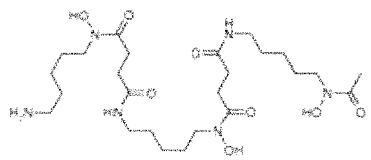

DFO-B (1g)

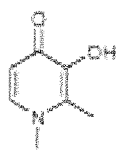

Deferiprone (1h)

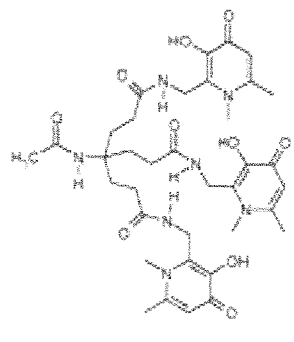

CP256 (1i)

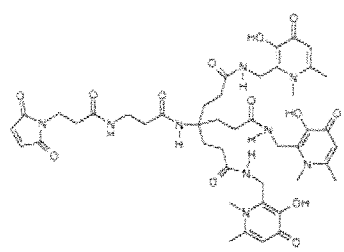

YM103 (1j)

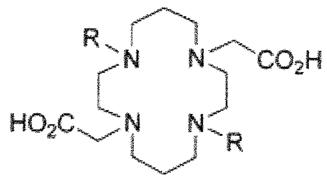

R =H TETA (1k)
R =$CH_2CO_2H$ TE2A (1l)

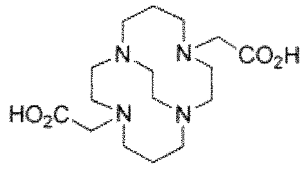

CB-TE2A (1m)

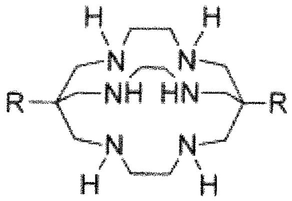

R =H Sar (1n)
R =$NH_2$ DiAmSar (1o)

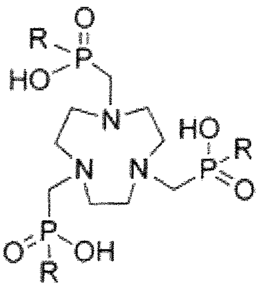

R =H TRAPH (1p)
R = $(CH2)_2CO_2H$ TRAP-Pr (1q)
R =$CH_2OH$ TRAP-OH (1r)
R =phenyl TRAP-Ph (1s)

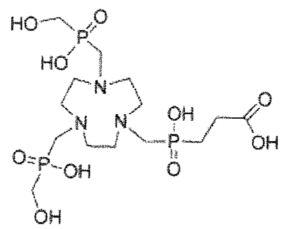

NOPO (1t)

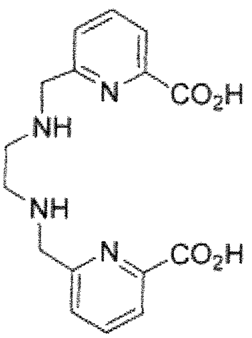

DEDPA (BCPE) (1u)

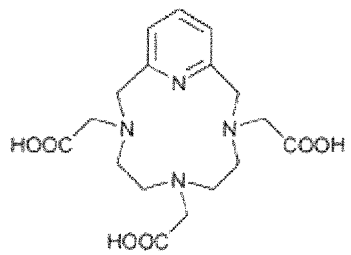

PCTA (1v)

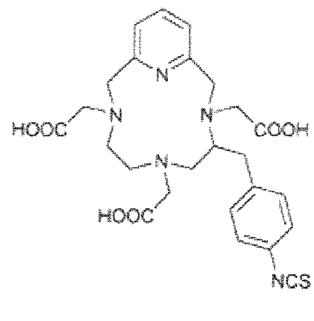

177 (1w)

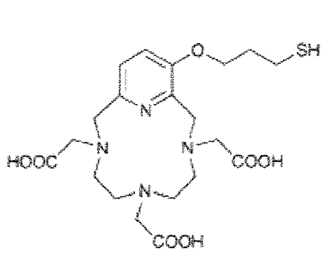

178 (1x)

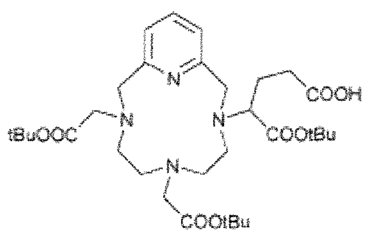

179 (1y)

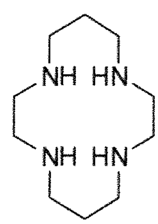

Cyclam (1z)

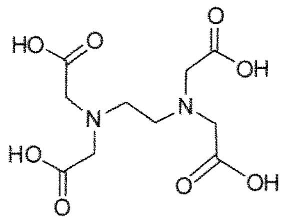

EDTA (1aa)

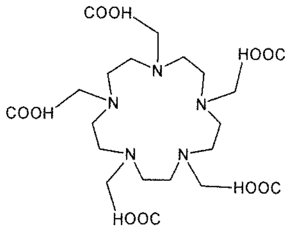

PEPA (1bb)

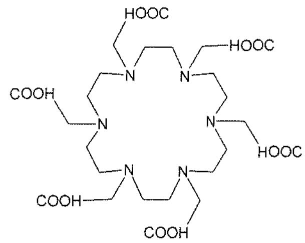

HEHA (1cc)

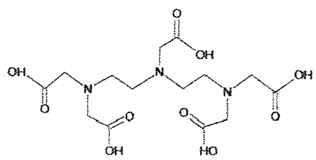

DTPA (1dd)

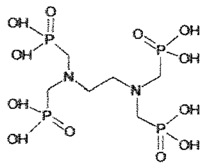

EDTMP (1ee)

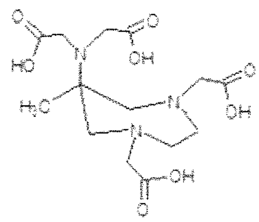

AAZTA (1ff)

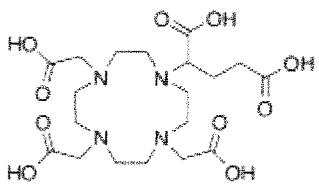

DOTAGA

DOTAGA (1gg)

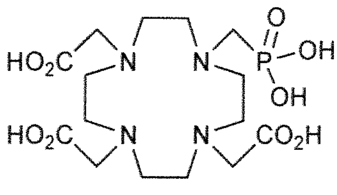

DO3AP(1hh)

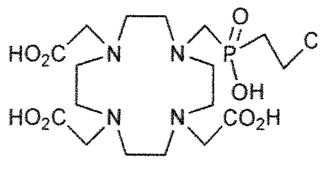

DO3AP$^{PrA}$ (1ii)

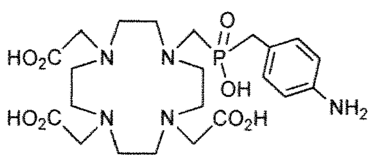

DO3AP$^{ABn}$ (1jj)

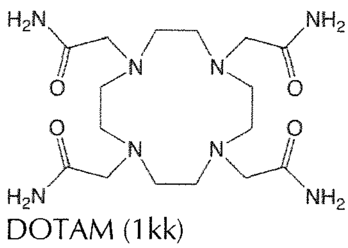

DOTAM (1kk)

More preferably, the chelating agent may be DOTA (1,4,7,10-tetraazacyclododecane-1,4,7,10-tetraacetic acid, which may be characterized by Formula (1a)), DOTAGA (2-[1,4,7,10-Tetraazacyclododecane-4,7,10-tris(acetate)]-pentanedioic acid, which may be characterized by Formula (1gg)), DOTAM (1,4,7,10-Tetrakis(carbamoylmethyl)-1,4,7,10-tetraazacyclododecane, which may be characterized by Formula (1 kk)) or derivatives thereof.

Other preferred chelators in the context of the present invention include (2-(4,7-bis(carboxymethyl)-1,4,7-triazonan-1-yl)-pentanedioic acid (NODAGA), 1,4,7-triazacyclononane-1,4,7-triacetic acid (NOTA), HBED-CC (N,N"-bis[2-hydroxy-5-(carboxyethyl)benzyl]ethylenediamine-N,N"-diacetic acid), 1,4,7-triazacyclononane phosphinic acid (TRAP), 1,4,7-triazacydo-nonane-1-[methyl(2-carboxyethyl)-phosphinic acid]-4,7-bis-[methyl(2-hydroxymethyl)-phosphinic acid] (NOPO), 3,6,9,15-tetra-azabicyclo[9,3,1]-pentadeca-1(15),11,13-triene-3,6,9-triacetic acid (PCTA), N'-{5-[Acetyl(hydroxy)amino]-pentyl}-N-[5-({4-[(5-aminopentyl)(hydroxy)amino]-4-oxobutanoyl}-amino)pentyl]-N-hydroxysuccinamide (DFO), and Diethylene-triaminepentaacetic acid (DTPA), and hydrazinonicotinamide (HYNIC).

Particularly preferably, the chelating agent is DOTA. Advantageously, DOTA effectively forms complexes with therapeutic (e.g. $^{90}Y$ or $^{177}Lu$) radionuclides. DOTA derivatives capable of complexing Scandium radionuclides ($^{43}Sc$,

$^{44}Sc$, $^{47}Sc$), including DO3AP (which may be characterized by Formula (1hh)), DO3AP$^{PrA}$ (which may be characterized by Formula (4ii)), or DO3AP$^{ABn}$ (which may be characterized by Formula (4jj)) may also be preferred and are described in Kerdjoudj et al. Dalton Trans., 2016, 45, 1398-1409.

The chelating agent, for example DOTA, may be complexed with any known radionuclide (in particular with the radionuclide as described above) as a central (metal) ion. Alternatively, the chelator group, for example DOTA, may not be complexed with a central (metal) ion, in particular a radionuclide as defined herein, and may thus be present in uncomplexed form. Should the chelator (e.g. DOTA) not be complexed with said metal ion, the carboxylic acid groups of the chelator can be in the form of a free acid, or in the form of a salt.

It is within the skill and knowledge of the skilled person in the art to select suitable combinations conjugates and radionuclides. In some embodiments, the chelator may be DOTA and the radionuclide may be $^{131}I$. In other embodiments, the chelator may be DOTA and the radionuclide may be $^{90}Y$. Particularly preferably, the chelator is DOTA and the radionuclide is $^{177}Lu$.

*Targeting molecule*

As used herein, the term "targeting molecule" (also referred to as "targeting moiety" refers to a molecule, which is able to bind (specifically) to a "target", such as a target cell (e.g., a cancer cell). In particular, the "target" may be a molecule located at the cell surface of a target cell (e.g., a cancer cell). Such a surface molecule, to which the targeting molecule binds, may be, for example, a receptor located at the surface of the cell. In particular, the surface molecule is specific for or overexpressed by the target cell (e.g., a cell "marker"). Accordingly, the targeting molecule is usually selected depending on the disease to be treated or diagnosed. In the context of a disease, e.g. cancer, the cells to be targeted with the radiolabeled complex, e.g. cancer cells, usually express specific molecules (or overexpress specific molecules), which may serve as "target" (surface molecule). The targeting molecule is typically selected such that it binds to said "targets" (surface molecules and, thus, target cells, e.g. cancer cells). The binding of the targeting molecule to the surface molecule may be reversible or irreversible.

According to the invention, the targeting molecule is selected from a peptide, a peptidomimetic, an antibody fragment, an antibody mimetic and folate.

Preferably, the targeting molecule is a peptide or polypeptide or a modified peptide or polypeptide.

Various surface molecules, to which the targeting molecule may suitably bind, are known in the art. In the following, examples of receptors and cell surface molecules present on tumor cells, which may be a target structure for the targeting molecule, are described in detail. However, the target structures are not limited to the receptors and cell surface molecules described below. Further receptors and cell surface molecules present on cancer or other disease cells are contemplated as target structures for the targeting molecules. Moreover, further targeting molecules targeting the receptors and cell surface molecules present on cancer or other disease cells are contemplated.

Among the targeting molecules which are described in the following, only those which are peptides, peptidomimetics, antibody fragments, antibody mimetics or folate can be used according to the invention.

PSMA-targeting compounds

Human Prostate-specific membrane antigen (PSMA) (also referred to as glutamate carboxypeptidase II (GCPII), folate hydrolase 1, folypoly-gamma-glutamate carboxypeptidase (FGCP), and N-acetylated-alpha-linked acidic dipeptidase I (NAALADase I)) is a type II transmembrane zinc metallopeptidase that is most highly expressed in the nervous system, prostate, kidney, and small intestine. It is considered as a tumor marker in prostate cancer. The term "Human Prostate-specific membrane antigen" or "PSMA" as used herein preferably refers to the protein encoded by the human FOLH1 gene. More preferably, the term refers to the protein as characterized under UniProt Acc. No. Q04609 (entry version 186, last modified May 10, 2017), or functional variants, isoforms, fragments or (post-translationally or otherwise modified) derivatives thereof.

The PSMA-binding targeting molecule may generally be a binding entity capable of selectively (and optionally irreversibly) binding to (human) Prostate-Specific Membrane Antigen (e.g., as described in Chang Rev Urol. 2004; 6(Suppl 10): S13-S18). The PSMA targeting molecule is preferably chosen by its ability to confer selective affinity towards PSMA. Preferred PSMA binding moieties are described in WO 2013/022797 A1, WO 2015/055318 A1 and EP 2862857 A1.

Accordingly, the PSMA targeting molecule may preferably be characterized by General Formula (2):

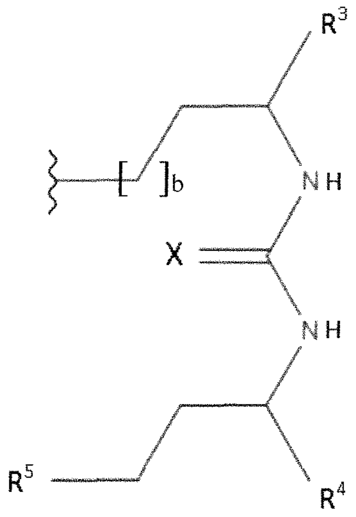

(2)

wherein

X is selected from O, N, 5 or P,
$R^3$, $R^4$ and $R^5$ are each independently selected from -COH, $-CO_2H$, $-SO_2H$, $-SO_3H$, $-SO_4H$, $-PO_2H$, $-PO_3H$, $-PO_4H_2$, $-C(O)-(C_1-C_{10})$alkyl, $-C(O)-O(C_1-C_{10})$alkyl, $-C(O)-NHR^8$, or $-C(O)-NR^8R^9$, wherein $R^8$ and $R^9$ are each independently selected from H, bond, (C1-C10)alkylene, F, Cl, Br, I, C(O), C(S), -C(S)-NH-benzyl-, -C(O)-NH-benzyl, $-C(O)-(C_1-C_{10})$alkylene, $-(CH_2)_p-NH$, $-(CH_2)_p-(C_1-C_{10})$alkyene, $-(CH_2)_p-NH-C(O)-(CH_2)_q$, $-(CH_rCH_2)_t-NH-C(O)-(CH_2)_p$, $-(CH_2)_p-CO-COH$, $-(CH_2)_p-CO-CO_2H$, $-(CH2)_p-C(O)NH-C[(CH_2)_q-COH]_3$, $-C[(CH_2)_p-COH]_3$, $-(CH2)_p-C(O)NH-C[(CH_2)_q-CO_2H]_3$, $-C[(CH_2)_p-CO_2H]_3$ or $-(CH_2)_p-(C_5-C_{14})$heteroaryl, and
b, p, q, r, t is each independently an integer selected from 0, 1, 2, 3, 4, 5, 6, 7, 8, 9, or 10.

In preferred PSMA targeting molecules, b may be an integer selected from 1, 2, 3, 4 or 5, $R^3$, $R^4$ and $R^5$ may each be $CO_2H$, X may be O.

Preferred examples of small-molecule PSMA targeting agents capable of binding to the extracellular domain of PSMA include, but are not limited to: radiolabeled N-[N-[(S)-1,3-dicarboxypropyl]carbamoyl]-S-[11C]methyl-l-cysteine (DCFBC), several urea-based peptidomimetic PSMA-inhibitors as described in Bouchelouche et al. Discov Med. 2010 Jan; 9(44): 55-61), including MIP-1095 (Hillier et al. Cancer Res. 2009 Sep 1;69(17):6932-40), and DOTA-conjugated PSMA-inhibitor PSMA-617 developed by Benešová et al (JNM 2015, 56: 914-920 and EP 2862 857 A1).

Urea-based PSMA ligands usually comprise three components: the binding motif (Glu-urea-Lys), a linker, and a radiolabel-bearing moiety (chelator molecule for radiolabeling or a prosthetic group for fluorinated agents). Examples of the most commonly used low-molecular-weight PSMA ligands are $^{123}$I-MIP-1072 and $^{123}$I-MIP-1095 (Barrett JA et al. J Nucl Med. 2013; 54:380-387; Zechmann et al., Eur J Nucl Med Mol Imaging. 2014; 41:1280-1292), chelator based PSMA-617 (Afshar-Oromieh A et al., J Nucl Med. 2015; 56:1697-1705) and PSMA-I&T (Weineisen M et al., J Nucl Med. 2015; 56:1169-1176), PSMA-I&S (Robu 5 et al., J Nucl Med. 2017; 58:235-242). As further $^{18}$F-labeled small-molecule urea derivatives $^{18}$F-DCFPyL (Chen Y et al., Clin Cancer Res. 2011; 17:7645-7653) and $^{18}$F-PSMA-1007 (Giesel FL et al., Eur J Nucl Med Molecular Imaging. 2017; 44:678-688) are mentioned.

Recently, Kelly et al. (Dual-Target Binding Ligands with Modulated Pharmacokinetics for Endoradiotherapy of Prostate Cancer. J Nucl Med. 2017 Sep;58(9):1442-1449. doi: 10.2967/jnumed.116.188722) evaluated agents exhibiting affinity for both PSMA and for human serum albumin (HSA). The ligands developed by Kelly et al. comprise a *p*-(iodophenyl)butyric acid entity for HSA binding and an urea-based PSMA binding entity. In the compounds developed by Kelly et al., radiotherapeutic iodine ($^{131}$I) is covalently attached to the HSA binding moiety, which is in turn directly connected to the PSMA binding entity via a hydrocarbyl chain.

Another example is a $^{177}$Lu-labeled phosphoramidate-based PSMA inhibitor with an albumin-binding entity (Choy et al. Theranostics 2017; 7(7): 1928-1939). A DOTA chelator complexing the $^{177}$Lu radionuclide was ether-linked to the irreversible PSMA inhibitor CTT1298 (EP 2970345 A1).

Thus, the targeting molecule in the radiolabeled complex is preferably a PSMA-targeting molecule, which may be bound to a chelator molecule, as defined above, and complexed with a radionuclide, as defined above, e.g. $^{177}$Lu.

The targeting molecule and the chelating agent usually form together conjugates or molecules (suitable for

radiolabeling). Various such conjugates/molecules are known in the art. Preferred conjugates comprising a chelating agent and a targeting molecule, which is able to bind to PSMA, are disclosed in WO 2018/215627 A1.

Preferred examples of conjugates comprising the targeting molecule and the chelating agent include PSMA-617 (shown in formula (3) below), PSMA-I&T (shown in formula (4) below) and Ibu-Dα-PSMA (shown in formula (5) below):

PSMA-617:

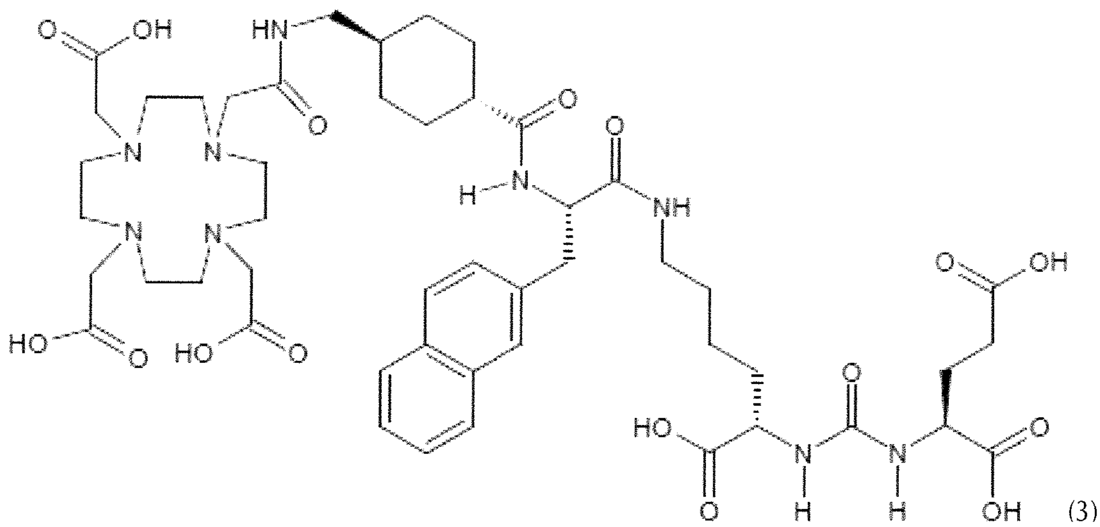

(3)

PSMA-I&T:

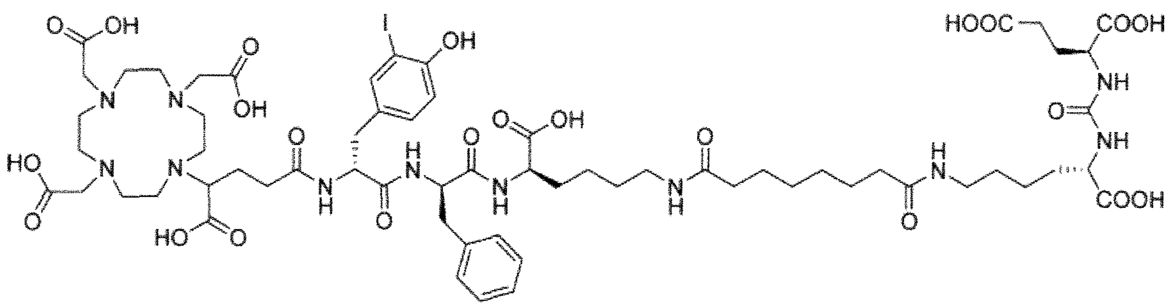

(4)

Ibu-Dα-PSMA:

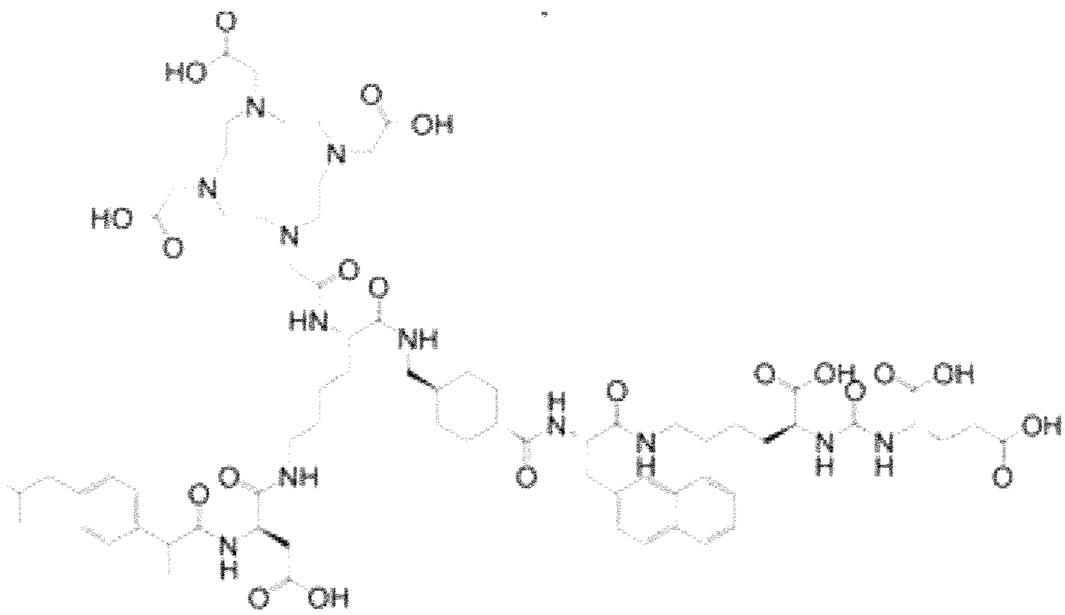

(5)

Somatostatin receptor targeting compounds

Other particularly suitable targeting molecules bind to a somatostatin receptor. Molecules binding to a somatostatin receptor are known in the art, such as somatostatin analogues.

Preferably, the targeting molecule is a somatostatin receptor binding peptide. More preferably said somatostatin receptor binding peptide is selected from octreotide, octreotate, lanreotide, vapreotide, pasireotide, ilatreotide, pentetreotide, depreotide, satoreotide, veldoreotide. Even more preferably, the targeting molecule is a somatostatin receptor binding peptide selected from octreotide and octreotate.

In particular for the treatment of well to moderately differentiated neuroendocrine tumors (NET), peptides targeting the somatostatin receptor (SSTR) may be used. In NET, radioligand therapy is well-established and may achieve high rates of long lasting tumor remission and stabilization. Peptides targeting the somatostatin receptor are e.g. somatostatin analogs tyr3-octreotide (D-Phe-c(Cys-Tyr-D-Trp-Lys-Thr-Cys)-Thr(ol)) and tyr3-octeotrate (D-Phe-c(Cys-Tyr-D-Trp-Lys-Thr-Cys)-Thr) (Capello A et al.: Tyr3-octreotide and Tyr3-octreotate radiolabeled with 177Lu or 90Y: peptide receptor radionuclide therapy results in vitro, Cancer Biother Radiopharm, 2003 Oct; 18(5): 761-8). Further examples of somatostatin receptor agonists are the peptides octreotide (D-Phe-cyclo(Cys-Phe-D-Trp-Lys-Thr-Cys)Thr(ol)), and NOC (D-Phe-cyclo(Cys-1-Nal-D-Trp-Lys-Thr-Cys)Thr(ol)).

Others examples of compounds targeting the somatostatin-receptor are somatostatin antagonistic peptides such as JR10 (p-$NO_2$-Phe-c(D-Cys-Tyr-D-Aph(Cbm)-Lys-Thr-Cys)D-Tyr-$NH_2$); JR11 (Cpa-c(D-Cys-Aph(Hor)-d-Aph(Cbm)-Lys-Thr-Cys)D-Tyr-NH2); BASS (p-$NO_2$-Phe-cyclo(D-Cys-Tyr-D-Trp-Lys-Thr-Cys)D-Tyr-$NH_2$; LM3 (p-Cl-Phe-cyclo(D-Cys-Tyr-D-Aph(Cbm)-Lys-Thr-Cys)D-Tyr-$NH_2$.

Preferred examples of (radio)pharmaceuticals based on somatostatin analogues include, but are not limited to: $^{177}$Lu-DOTATOC ($^{177}$Lu-DOTA°-[Tyr3]-octreotide) ($^{177}$Lu-DOTA-D-Phe-cyclo(Cys-Tyr-D-Trp-Lys-Thr-Cys]-Thr(ol), $^{177}$Lu-DOTANOC ($^{177}$Lu-DOTA-D-Phe-cyclo(Cys-1-Nal-D-Trp-Lys-Thr-Cys)Thr(ol)), $^{177}$Lu-DOTATATE ($^{177}$Lu-DOTA-D-Phe-cyclo(Cys-Tyr-D-Trp-Lys-Thr-Cys)Thr), $^{68}$Ga-DOTATOC ($^{68}$Ga-DOTA-D-Phe-cyclo(Cys-Tyr-D-Trp-Lys-Thr-Cys)Thr(ol)), $^{68}$Ga-DOTANOC ($^{68}$Ga-DOTA-D-Phe-cyclo(Cys-1-Nal-D-Trp-Lys-Thr-Cys)Thr(ol)), $^{90}$Y-DOTATOC ($^{90}$Y-DOTA-D-Phe-cyclo(Cys-Tyr-D-Trp-Lys-Thr-Cys)Thr(ol)), $^{90}$Y-DOTATATE ($^{90}$Y-DOTA-D-Phe-cyclo(Cys-Tyr-D-Trp-Lys-Thr-Cys)Thr), $^{111}$In-DTPA-octreotide ($^{111}$In-DTPA-D-Phe-cyclo(Cys-Phe-D-Trp-Lys-Thr-Cys)Thr(ol)).

Further examples of (radio)pharmaceuticals based on somatostatin analogues include, but are not limited to:: $^{111}$In-DOTA-BASS ($^{111}$In-DOTA-p-$NO_2$-Phe-cyclo-(D-Cys-Tyr-D-Trp-Lys-Thr-Cys)D-Tyr-$NH_2$, $^{111}$In-DOTA-JR11 ($^{111}$In-DOTA-Cpa-cyclo[D-Cys-Aph(Hor)-D-Aph(Cbm)-Lys-Thr-Cys]D-Tyr-$NH_2$), $^{68}$Ga-DOTA-JR11 (Ga-OpS201) ($^{68}$Ga-DOTA-Cpa-cyclo[D-Cys-Aph(Hor)-D-Aph(Cbm)-Lys-Thr-Cys]D-Tyr-$NH_2$), $^{68}$Ga-DODAGA-JR11 (Ga-OPS202) ($^{68}$Ga-NODAGA-Cpa-cyclo[D-Cys-Aph(Hor)-D-Aph(Cbm)-Lys-Thr-Cys]D-Tyr-$NH_2$), $^{177}$Lu-DOTA-JR11 (Lu-OPS201) ($^{177}$Lu-DOTA-Cpa-cyclo[D-Cys-Aph(Hor)-D-Aph(Cbm)-Lys-Thr-Cys]D-Tyr-$NH_2$).

Thus, the targeting molecule in the radiolabeled complex is preferably a somatostatin receptor targeting molecule, which may be bound to a chelator molecule, as defined above, and complexed with a radionuclide, as defined above, e.g. $^{177}$Lu.

Preferred conjugates comprising a chelating agent and a targeting molecule, which is able to bind to a somatostatin receptor, include DOTA-OC ([DOTA$^0$,$_D$-Phe$^1$]octreotride), DOTATOC ([DOTA$^0$,$_D$-Phe',Tyr$^3$]octreotride; INN: edotreotide), DOTANOC ([DOTA$^0$,$_D$-Phe$^1$,$_1$-Nal$^3$]octreotride), DOTATATE ([DOTA$^0$,$_D$-Phe$^1$,Tyr$^3$]octreotate; INN: oxodotreotide), DOTALAN ([DOTA$^0$,$_D$-β-Nal$^3$]octreotride), DOTAVAP ([DOTA$^0$,$_D$-Phe$^1$,Tyr$^3$]vapreotide), satoreotide trizoxetan and satoreotide tetraxetan. More preferably, the molecule comprising a chelating agent and a targeting molecule selected from DOTATOC and DOTATATE.

Accordingly, the radiolabeled complex preferably comprises or consists of (i) the radionuclide and (ii) DOTATOC or DOTATATE. Particularly preferably, the radiolabeled complex (comprising the radionuclide, the targeting molecule and the chelating agent) is $^{177}$Lu-DOTATOC ($^{177}$Lu-edotreotide) or $^{177}$Lu-DOTATATE ($^{177}$Lu- oxodotreotide).

Folate conjugates

Folate receptor (FR)-α attracted most interest as a tumor-associated target for targeted therapy concepts. Targeting of FR-positive tumor cells *in vitro* and *in vivo* has been exemplified by a number of research groups using folic acid conjugates with a variety of therapeutic probes. The FR has thus proven a valuable target for nuclear imagine using folic acid radioconjugates.

However, using folate-based radiopharmaceuticals for therapy has long been regarded as an unattainable goal because of their considerable renal accumulation. However, the combination of the radiolabeled complex with PAH allows to reduce off-site accumulation of the radiopharmaceuticals *in vivo,* thus improving the tumor-to-kidney ratios.

Preferred examples of folate conjugate radiopharmaceuticals use $^{99m}$Tc (Guo et al., J Nucl Med. 1999; 40: 1563-1569; Mathias et al., Bioconjug Chem. 2000; 11:253-257; Leamon et al., Bioconjug Chem. 2002; 13:1200-1210; Reddy et al., J Nucl. Med. 2004; 45:857-866; Müller et al., J Nucl Med Mol Imaging 2006; 33:1007-1016; Müller et al., Bioconjug Chem. 2006; 17:797-806), $^{111}$In (Siegel et al., J Nucl Med. 2003; 44:700-707), $^{66/67/68}$Ga (Mathias et al., Nucl Med Biol. 1999; 26:23-25; Mathias et al., Nucl Med Biol. 2003; 30:725-731) and $^{18}$F (Bettio et al., J Nucl Med. 2006; 47:1153-1160).

Representative folate conjugates are e.g. $^{111}$In-DTPA-folate, $^{177}$Lu-EC0800, $^{177}$Lu-cm09, $^{149/161}$Tb-cm09, $^{99m}$Tc(CO)$_3$, $^{99m}$Tc-EC20, $^{111}$In-DTPA-folate, $^{111}$In/$^{177}$Lu-DOTA-click-folate, $^{67}$Ga-DOTA-Bz-folate ($^{67}$Ga-EC0800), $^{68}$Ga-NODAGA-folate and the complex shown in below formula (6):

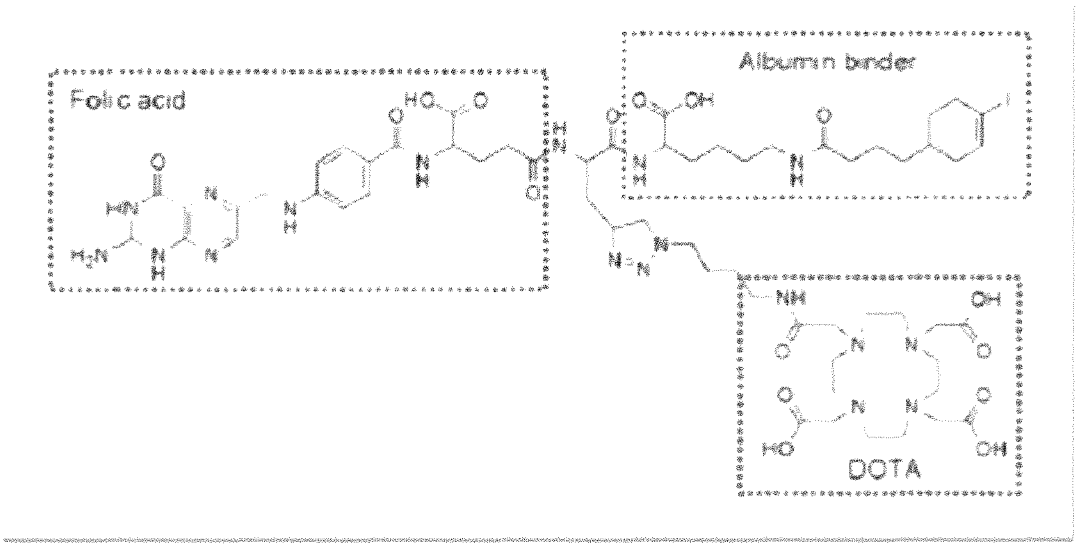


(6)

CCK2 receptor-targeting compounds

PAH or a salt or carboxylate derivative thereof can also be suitably used in combination with radiopharmaceuticals targeting the CCK2 receptor.

The CCK2 receptor (cholecystokinin) is located in areas of the central and peripheral nervous system and is overexpressed in several types of human cancer, as medullar thyroid carcinomas, small cell lung cancers and stromal ovarian carcinomas. Research has been done on developing suitable radioligands for targeting the CCK2-receptor *in vivo.* A variety of radiolabeled CCK/gastrin-related peptides has been synthesized and characterized. All peptides have the C-terminal CCK receptor-binding tetrapeptide sequence Trp-Met-Asp-Phe-$NH_2$ in common or derivatives thereof. The peptides can be categorized based on the sequence of their parent peptide (gastrin or CCK) and on their form (i.e.

linear, cyclic, multimers).

Examples for CCK receptor ligands are gastrin analogs, such as Sargastrin (Gln-Gly-Pro-Trp-Leu-Glu-Glu-Glu-Glu-Glu-Ala-Tyr-Gly-Trp-Nle-Asp-Phe-$NH_2$), Minigastrin 0 (MG-0) $_D$-Glu-$(Glu)_5$-Ala-Tyr-Gly-Trp-Met-Asp-Phe-$NH_2$), Minigastrin 11 (MG-11) ($_D$-Glu-Ala-Tyr-Gly-Trp-Met-Asp-Phe-$NH_2$), cyclo-Minigastrin 1 (cyclo-MG1) (cyclo[γ-D-Glu-Ala-Tyr-D-Lys]-Trp-Met-Asp-Phe-$NH_2$), cyclo-Minigastrin 2 (cyclo-MG2) (cyclo[γ-D-Glu-Ala-Tyr-D-Lys]-Trp-Nle-Asp-Phe-$NH_2$, Demogastrin 1 ($_D$-Glu-$(Glu)_5$-Ala-Tyr-Gly-Trp-Met-Asp-Phe-$NH_2$), Demogastrin 2 ($_D$-Glu-$(Glu)_5$-Ala-Tyr-Gly-Trp-Met-Asp-Phe-$NH_2$, H2-Met (His-His-Glu-Ala-Tyr-Gly-Trp-Met-Asp-Phe-$NH_2$), H2-Nle (His-His-Glu-Ala-Tyr-Gly-Trp-Nle-Asp-Phe-$NH_2$), H6-Met $(His)_6$-Glu-Ala-Tyr-Gly-Trp-Met-Asp-Phe-$NH_2$); and CCK8 analogs, such as CCK8 ($_D$-Asp-Tyr-Met-Gly-Trp-Met-Asp-Phe-$NH_2$), CCK8(Nle) (D-Asp-Tyr-Nle-Gly-Trp-Nle-Asp-Phe-$NH_2$), sCCK8 (D-Asp-Tyr($OSO_3H$)-Met-Gly-Trp-Met-Asp-Phe-$NH_2$), sCCK8[$Phe^2$(*p*-$CH_2SO_3H$), $Nle^{3,6}$] ($_D$-Asp-Phe(*p*-$CH_2SO_3H$)-Nle-Gly-Trp-Nle-Asp-Phe-$NH_2$), sCCK8[$Phe^2$(*p*-$CH_2SO_3H$), $HPG^{3,6}$] ($_D$-Asp-Phe(*p*-$CH_2SO_3H$)-HPG-Gly-Trp-HPG-Asp-Phe-$NH_2$).

The CCK receptor targeting peptides are preferably radiolabeled with the radionuclides for imaging or therapeutic applications. Suitable radionuclides comprise the radionuclides specified above, and in particular comprise the radionuclides $^{99m}$Tc, $^{111}$In, $^{18}$F, $^{68}$Ga, $^{131}$1, $^{90}$Y, and $^{177}$Lu. To allow radiolabeling with a radionuclide, a chelator conjugated to the peptide is preferably used. As a chelator, the chelators specified above can be used, wherein DOTA, DOTAGA, DOTAM, DTPA and HYNIC are preferred.

Accordingly, the radiolabeled complex may include a CCK2 receptor targeting molecule, such as $^{177}$Lu-DOTA-Sargastrin, $^{111}$In-DTPA-MGO, $^{111}$In-DOTA-MG11, $^{111}$In-DOTA-MG11(Nle), $^{111}$In-DOTA-H2-Met, $^{111}$In-DOTA-H2-Nle, $^{111}$In-DOTA-H6-Met, $[^{99m}Tc]_2N_4{}^0$, $_D$-Glu'-MG ($^{99m}$Tc-Demogastrin 1), $[^{99m}Tc]_2N_4{}^{0-1}$,$Gly^0$,D-$Glu^1$-MG ($^{99m}$Tc-Demogastrin 2), $^{99m}$Tc-HYNIC-MG11, $^{99m}$Tc-HYNIC-cyclo-MG1, $^{99m}$Tc-HYNIC-cyclo-MG2; and CCK8 analogs, such as $^{111}$In-DTPA-CCK8, $^{111}$In-DTPA-CCK8(Nle), $^{99m}$Tc-HYNIC-CCK8, $^{99m}$Tc-HYNIC-sCCK8, $^{111}$In-DOTA-sCCK8[$Phe^2$(*p*-$CH_2SO_3H$), $Nle^{3,6}$], and $^{111}$In-DOTA-sCCK8[$Phe^2$(*p*-$CH_2SO_3H$), $HPG^{3,6}$].

Integrin-binding molecules

PAH or a salt thereof can also be suitably used in combination with radiopharmaceuticals targeting integrins.

Integrins are heterodimeric glycoproteins consisting of an $\alpha$- and $\beta$-subunit. There are 24 different combinations of the eight $\beta$-units and the eighteen $\alpha$-units known. The integrins mediate cell-cell and cell-matrix interactions and transduce signals across the plasma membrane via insight-out and outside-in signaling. Some of the integrins play an important role during migration of endothelial as well as tumor cells during tumor-induced angiogenesis and tumor metastasis. Angiogenesis, the formation of new blood vessels out of the preexisting vasculature, is a critical step in the development and dissemination of various human tumors. A variety of therapeutic strategies in oncology are focused on the inhibition of tumor-induced angiogenesis. Concerning the integrins, significant attention has been paid to the role of integrin $\alpha V\beta 3$ and $\alpha V\beta 5$, as they are prominent on proliferating vascular endothelial cells.

Thus, one of the most prominent target structures used for the development of radiopharmaceuticals for imaging angiogenesis is the integrin $\alpha V\beta 3$.

Tumor-induced angiogenesis can be blocked *in vivo* by antagonizing the $\alpha_v\beta_3$ integrin with small peptides containing the Arg-Gly-Asp (RGD) amino acid sequence. This tripeptidic sequence, naturally present in extracellular matrix proteins, is the primary binding site of the $\alpha_v\beta_3$ integrin. Because of selective expression of $\alpha_v\beta_3$ integrin in tumors, radiolabeled RGD peptides are attractive candidates for $\alpha_v\beta_3$ integrin targeting in tumors. Over the last decade, many radiolabeled linear and cyclic RGD peptides have been evaluated as radiotracers for imaging tumors by SPECT or PET, as well as therapeutic agents.

PAH or a salt thereof can also be suitably used in combination with radiopharmaceuticals comprising radiolabeled RGD peptides.

Suitable radionuclides comprise the radionuclides specified above, and in particular comprise the radionuclides $^{18}$F, $^{99m}$Tc, $^{68}$Ga, $^{111}$In, $^{131}$I, $^{90}$Y, $^{67}$Cu, and $^{177}$Lu. To allow radiolabeling with a radionuclide, a chelator conjugated to the peptide is preferably used. As a chelator, any suitable the chelators, e.g. as specified above, can be used, wherein NOTA, DOTA, DOTAGA, DOTAM, DTPA, HYNIC are preferred.

For example, PAH or a salt or carboxylate derivative thereof can also be suitably used in combination with $^{18}$F-Galacto-RGD, $^{99m}$Tc-NC100692 ($^{99m}$Tc-maracilatide), $^{18}$F-AH11185 ($^{18}$F-Fluciclatide), $^{18}$F-RGD-K5, $^{68}$Ga-NOTA-RGD, $^{18}$F-FPPRGD2, $^{18}$F-AIF-NOTA-PRGD2 ($^{18}$F-Alfatide), $^{18}$F-NOTA-E[PEG4-c(RGDfk)]$_2$ ($^{18}$F-Alfatide II), $^{68}$Ga-NOTA-PRGD2, $^{67}$Cu-cyclam-RAFT-c(-RGDfK-)$_4$, $^{111}$In-DOTA-E-[c(RGDfK)]$_2$, $^{99m}$Tc-HYNIC-E-[c(RGDfK)]$_2$.

Neurotensin receptor-targeting compounds

Neurotensin receptor 1 (NTR1) is overexpressed in ductal pancreatic adenocarcinoma, which is one of the deadliest cancers. Several NTR1 antagonists have been developed, such as SR142948A and SR48692, and $^{177}$Lu-

3BP-2273, which is a $^{177}$Lu-labeled DOTA-conjugated NTR1 antagonist that has been developed on the basis of SR142948A. It has been used for the treatment of ductal pancreatic adenocarcinoma (Baum RP et al., The Journal of Nuclear Medicine, Vol. 59, No. 5, May 2018).

Therefore, PAH or a salt or carboxylate derivative thereof can also be suitably used in combination with radiopharmaceuticals targeting the Neurotensin receptor 1, in particular of radiolabeled NTR1 antagonists for cancer diagnosis or therapy, preferably $^{177}$Lu- or $^{68}$Ga-labeled NTR1 antagonists, more preferably $^{177}$Lu-3BP-2273, even though other radionuclides, for example the radionuclides mentioned above, as well as other chelators, for example the chelators mentioned above, may be contemplated.

Glucagon-like peptide-1 (GLP-1) receptor targeting compounds

The GLP-1 receptor is overexpressed on essentially all benign insulinomas and also on gastrinomas. Benign insulinomas which emerge from β-cells of the pancreas and are present as small nodules, secrete insulin leading to potentially life-threatening hypoglycemia.

Therefore, PAH or a salt thereof can also be suitably used in combination with radiopharmaceuticals targeting the GLP-1 receptor. Non-limiting examples thereof include $^{111}$In-, $^{99m}$Tc-, and $^{68}$Ga-labeled peptides based on the 39-mer peptide exendin-4, such as Lys$^{40}$(Ahx-DOTA-$^{111}$In)$NH_2$-extendin-4, for example. However, other radionuclides, for example the radionuclides mentioned above, as well as other chelators, for example the chelators mentioned above, may be contemplated.

Gastrin releasing peptide (GRP) receptor targeting compounds

PAH or a salt thereof can also be suitably used in combination with radiopharmaceuticals targeting the GRP receptor.

GRP receptors have been demonstrated in major human tumors, such as breast cancer and prostate cancer. Bombesin is a tetradecapeptide neurohormone and an amphibian homolog of mammalian GRP (a 27mer peptide). Several bombesin analogs and bombesin antagonists have been developed and labeled with different radioisotopes (e.g. $^{68}$Ga, $^{64}$Cu, $^{18}$F) using different chelators. Examples thereof include a pan-bombesin analog $^{68}$Ga-BZH3 (Zhang H et al., Cancer Res 2004; 64: 6707-6715), and a $^{177}$Lu-labeled bombesin(7-14) derivative coupled to DOTA via a Gly-4-aminobenzoyl spacer (Bodei L et al., Eur J Nucl Med Mol Imaging 2007: 34(suppl 2): S221).

However, PAH or a salt thereof can also be suitably used in combination with radiopharmaceuticals targeting the GRP receptor comprising other radionuclides, for example the radionuclides mentioned above, as well as other chelators, for example the chelators mentioned above.

Neurokinin type 1 receptor targeting compounds

The neurokinin type 1 receptor is consistently overexpressed on glioma cells and on tumor vessels (Hennig IM et al., Int J Cancer 1995; 61: 786-792). The radiolabeled 11-amino-acid peptide substance P (Arg Pro Lys Pro Gln Gln Phe Phe Gly Leu Met) acting via the neurokinin type 1 receptor can suitably be used to target malignant gliomas. In particular, substance P has been conjugated to the chelator DOTAGA, and $^{90}$Y-labeled DOTAGA-substance P has been used in clinically studies (Kneifel 5 et al., Eur J Nucl Med Mol Imaging. 2007; 34: 1388-1395. In another study, the feasibility and effectiveness of targeted α-radionuclide therapy for brain tumors was assessed using the α-radiation-emitting conjugate 213Bi-DOTA-[THi8,Met(O2)11]-substance P (Cordier et al., Eur J Nucl Med Mol Imaging. 2010; 37: 1335-1344).

Therefore, PAH or a salt thereof can also be suitably used in combination with radiopharmaceuticals targeting the neurokinin type 1 receptor, in particular substance P conjugates (comprising a radionuclide, and a chelator coordinating the radionuclide).

Affilins

PAH or a salt thereof can also be suitably used in combination with radiopharmaceuticals comprising antibody mimetics.

Affilins are artificial proteins designed to selectively bind antigens. Affilin proteins are structurally derived from human ubiquitin or gamma-B crystallin, respectively. Affilin proteins are constructed by modification of surface-exposed amino acids of these proteins and isolated by display techniques such as phage display and screening. They resemble antibodies in their affinity and specificity to antigens but not in structure, which makes them a type of antibody mimetic. Affilin® was developed by Scil Proteins GmbH as potential biopharmaceutical drugs, diagnostics and affinity ligands. Affilin molecules can be easily modified and are suitable to kill tumor cells specifically by irradiation.

Multispecific Affilin molecules can be generated, binding different targets simultaneously. Radionuclides or cytotoxins can be conjugated to Affilin proteins, making them potential tumor therapeutics and diagnostics. Radionuclide-chelator-Affilin conjugates, e.g. $^{177}$Lu-DOTA-Affilin, have been designed for therapy purposes. PAH or a salt thereof can also be suitably used in combination with these Affilin conjugates. It may also be used in combination with further Affilin conjugates comprising other radionuclides (for example as specified above) and chelators (for example as specified above), respectively.

Particularly suitable surface molecules are PSMA and a somatostatin receptor targeted by the targeting molecule of the radiolabeled complex. Accordingly, the targeting molecule is preferably able to bind to PSMA or a somatostatin receptor, e.g. as described above.

The targeting molecule may be either directly or indirectly (e.g., by using linkers or spacers) linked to the chelating agent. The linking bond(s) is/are covalent or non-covalent bond(s) between the targeting molecule, optionally the linker or spacer, and the chelating agent.

Preferably the bond(s) is/are covalent. Preferably, the radiolabeled complex comprises linkers. Particularly suitable linkers and spacers are described in WO 2018/215627 A1 and in WO 2020/109523 A1.

**Para-aminohippuric acid (PAH)**

Aminohippuric acid or *para*-aminohippuric acid (PAH), a derivative of hippuric acid, is an amide derivative of the amino acid glycine and *para*-aminobenzoic acid that is not naturally found in humans. They are covalently linked by an amide bond. The structural formula of para-aminohippuric acid (PAH) is shown in formula (7):

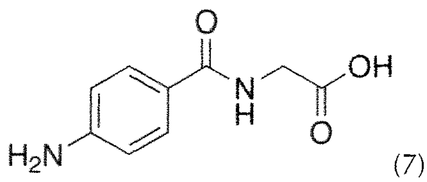

(7)

PAH's sodium salt, aminohippurate sodium, is a known diagnostic agent which is widely used in diagnostic testing of the kidney function, in particular for measuring renal plasma flow. The structural formula of sodium aminohippurate (sodium para-aminohippurate) is shown in formula (8):

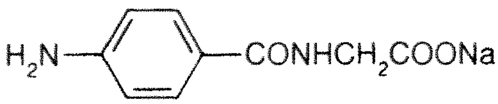

(8)

As used herein, the terms "aminohippuric acid", "para-aminohippuric acid" and "PAH" are used synonymously and generally refer to para-aminohippuric acid and salts thereof (aminohippurate salt, in particular alkali or earthalkali salt, such as the sodium salt), unless specifically stated otherwise.

Typically, PAH is provided as a sterile, non-preserved 20 % aqueous solution for injection. PAH is generally well tolerated and does essentially not exhibit any side effects. It is of negligible toxicity (the intravenous LD50 in female mice is 7.22 g/kg). Phenomena like vomiting and nausea or hyperkalemia are not or, if at all, rarely reported only.

Preferably, the combination of the present invention comprises (a) the radiolabeled complex as described herein; and (b) para-aminohippuric acid, but preferably not a salt of para-aminohippuric acid.

More preferably, the combination of the present invention comprises (a) the radiolabeled complex as described herein; and (b) a salt of para-aminohippuric acid, but preferably not para-aminohippuric acid.

Preferred salts of PAH include alkali or earthalkali salts of PAH. The sodium salt of PAH, sodium aminohippurate, is particularly preferred. It is understood that the salt of PAH is usually a pharmaceutically acceptable salt of PAH, in particular a salt which is not toxic when administered at an effective dose.

Preferably, PAH or a salt thereof, preferably aminohippurate sodium, is comprised in a buffered aqueous solution, e.g. an isotonic or hypertonic solution, e.g. in water for injection (WFI). Particularly preferably, PAH is provided in a solution for injection, e.g. a 10% (w/v) PAH, in particular sodium aminohippurate, solution for injection or a 20% (w/v) PAH, in particular sodium aminohippurate, solution for injection. Such PAH solutions for injection are commercially available. The PAH solution for injection may comprise PAH, in particular sodium aminohippurate, (but preferably no further active ingredients) and water for injection.

In the present invention, PAH or a salt thereof, preferably aminohippurate sodium, is used in an amount which

is sufficient to effectively improve the anti-tumor effects of the radiolabeled complex. The effective amount of PAH may be determined by routine experiments, e.g. by using animal models. Such models include, without implying any limitation, rabbit, sheep, mouse, rat, dog and non-human primate models.

For instance, the administered amount of PAH may range (per kg body weight) from about 0.1 mg/kg to 10 g/kg, preferably from about 0.5 mg/kg to 5 g/kg, more preferably from about 1 mg/kg to 1 g/kg.

Preferably, PAH or a salt thereof, preferably aminohippurate sodium, is used in an amount of about 5 mg to about 500 mg per kilogram of body weight, for example in an amount of about 5, 10, 20, 30, 40, 50, 60, 70, 80, 90, 100, 150, 200, 250, 300, 350, 400, 450, 500 mg per kilogram of body weight up to 500 mg per kilogram of body weight. More preferably, PAH or a salt thereof, preferably aminohippurate sodium, is used in an amount of about 50 mg to about 500 mg per kilogram of body weight, more preferably, from about 50 mg to about 250 mg per kilogram of body weight, for example in an amount of about 50, 55, 60, 65, 70, 75, 80, 85, 90, 95, 100, 110, 120, 130, 140, 150, 160, 170, 180, 190, 200, 210, 220, 230, 240 or 250 mg per kilogram of body weight. Even more preferably, PAH or a salt thereof, preferably aminohippurate sodium, is used in an amount of about 75 mg to about 200 mg per kilogram of body weight, for example in an amount of about 75, 80, 85, 90, 95, 100, 105, 110, 115, 120, 125, 130, 135, 140, 145, 150, 155, 160, 165, 170, 175, 180, 185, 190, 195, up to 200 mg per kilogram of body weight or 200 mg per kilogram of body weight. Still more preferably, PAH or a salt thereof, preferably aminohippurate sodium, is used in an amount of about 80 mg to about 160 mg per kilogram of body weight, for example in an amount of about 80, 85, 90, 95, 100, 105, 110, 115, 120, 125, 130, 135, 140, 145, 150, 155, up to 160 mg per kilogram of body weight, or 160 mg per kilogram of body weight.

PAH or the salt thereof is preferably used in a larger (molar and/or w/w) quantity than the (co-administered) radiolabeled complex as described herein.

In some embodiments, the radiolabeled complex and PAH (or a salt thereof, preferably aminohippurate sodium) are used in a ratio of about 1/1.000.000 to 1/10 (w/w), preferably of about 1/500.000 to 1/100 (w/w), more preferably from about 1/250.000 to about 1/500 (w/w).

Preferably, the radiolabeled complex and PAH (or a salt thereof, preferably aminohippurate sodium) are used in a ratio of about 1/250.000 to about 1/5.000 (w/w), for example in a ratio of about 1/250.000, 1/200.000, 1/150.000, 1/100.000, or 1/50.000 to about 1/5.000 (w/w), more preferably in a ratio of about 1/240.000 to about 1/8.000 (w/w), for example in a ratio of about 1/240.000, 1/230.000, 1/220.000, 1/210.000, 1/200.000, 1/190.000, 1/180.000, 1/170.000, 1/160.000, 1/150.000, 1/140.000, 1/130.000, 1/120.000, 1/110.000, 1/100.000, 1/90.000, 1/80.000, 1/70.000, 1/60.000, 1/50.000, 1/40.000, 1/30.000, 1/20.000, 1/19.000, 1/18.000, 1/17.000, 1/16.000, 1/15.000, 1/14.000, 1/13.000, 1/12.000, 1/11.000, 1/10.000, 1/9.000, or 1/8.000 (w/w). It is also preferred that the radiolabeled complex and PAH (or a salt thereof, preferably aminohippurate sodium) are used in a ratio of about 1/100.000 to about 1/10.000 (w/w), for example in a ratio of about 1/100.000, 1/95.000, 1/90.000, 1/85.000, 1/80.000, 1/75.000, 1/70.000, 1/65.000, 1/60.000 1/55.000, 1/50.000, 1/45.000, 1/40.000, 1/35.000, 1/30.000, 1/25.000, 1/20.000, 1/15.000, 1/10.000 (w/w). It is also preferred that the radiolabeled complex and PAH (or a salt thereof, preferably aminohippurate sodium) are used in a ratio of about 1/50.000 to about 1/40.000 (w/w), for example in a ratio of about 1/50.000, 1/49.000, 1/48.000, 1/47.000, 1/46.000, 1/45.000, 1/44.000, 1/43.000, 1/42.000, 1/41.000, 1/40.000 (w/w).

Medical treatment and uses

The present invention relates to the combination as described above for use in the treatment of cancer, wherein the combined administration of the radiolabeled complex and para-aminohippuric acid or a salt thereof increases the anti-tumor efficacy of the radiolabeled complex.

In some embodiments, the subject is a human being, in particular a human cancer patient. The human being may be a human being of the age of 18 or older.

In general, radionuclide therapy may be applied to any cancer that satisfies the targeting criteria needed for delivery of radionuclides. As the combined administration of PAH and the radiolabeled complex increases the anti-tumor efficacy of the radiolabeled complex, it can be applied to any radionuclide cancer therapy. Therefore, the principle of the combined use of a radiolabeled complex and PAH is not restricted to certain kinds of cancer.

In general, the skilled person usually selects the targeting molecule in view of the cancer to be treated, such that the radiolabeled complex is delivered by the targeting molecule to the target cancer cells.

For example, radiolabeled complexes with a targeting molecule binding to PSMA, may be used in the treatment of any cancer expressing PSMA. In particular, the presence of PSMA-expressing cells or tissues may be indicative of a prostate tumor (cell), a metastasized prostate tumor (cell), a renal tumor (cell), a pancreatic tumor (cell), a bladder tumor (cell), and combinations thereof. Accordingly, the cancer to be treated is preferably prostate cancer, pancreatic cancer, renal cancer or bladder cancer.

For example, radiolabeled complexes with a targeting molecule binding to a somatostatin receptor, in particular somatostatin receptor 2 (SSTR-2) or somatostatin receptor 5 (SSTR-5), more particularly somatostatin receptor 2 (SSTR-2), may be used in the treatment of any cancer expressing a somatostatin receptor. Accordingly, the cancer is preferably

a neuroendocrine tumor (NET). In particular, the NET may be selected from the group consisting of gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, small cell lung cancer (SCLC), prostate cancer, melanoma, meningioma, glioma, medulloblastoma, hemangioblastoma, supratentorial primitive, neuroectodermal tumor, and esthesioneuroblastoma. Further non-limiting examples of NET tumors include functional carcinoid tumor, insulinoma, gastrinoma, vasoactive intestinal peptide (VIP) oma, glucagonoma, serotoninoma, histaminoma, ACTHoma, pheocromocytoma, and somatostatinoma.

In some embodiments, the human being to be treated suffers from a tumor, which has been been diagnosed as SSTR-2 positive.

In some embodiments, the NET may be selected from the group consisting of carcinoid tumor, pheochromocytoma, paraganglioma, medullary thyroid cancer, pulmonary neuroendocrine tumor, thymic neuroendocrine tumor, a carcinoid tumor, pituitary adenoma, adrenal gland tumors, Merkel cell carcinoma, breast cancer, Non-Hodgkin lymphoma, Hodgkin lymphoma, head & neck tumor, urothelial carcinoma (bladder), renal Cell Carcinoma, hepatocellular Carcinoma, GIST, neuroblastoma, bile duct tumor, cervix tumor, Ewing sarcoma, osteosarcoma, small cell lung cancer (SCLC), prostate cancer, melanoma, meningioma, glioma, medulloblastoma, hemangioblastoma, supratentorial primitive, neuroectodermal tumor, and esthesioneuroblastoma.

In general, preferred types of cancers to be treated include those, for which radionuclide therapy is established and currently under investigation. Usually, the type of cancer investigated primarily reflects developments related to the available targets (and targeting molecules for the specific delivery of the radionuclide to the cancer cells). Preferred examples of cancers include, but are not limited to, thyroid malignancies, haematological malignancies, hepatic malignancies, prostate cancer, colorectal cancer, breast cancer, neuroendocrine and somatostatin receptor cancers. In some embodiments, the cancer is selected from neuroendocrine tumors, prostate cancer, pancreatic cancer, renal cancer, bladder cancer, brain cancer, gastrointestinal cancer, medullar thyroid carcinomas, small or non-small cell lung cancers, stromal ovarian carcinomas, ductal pancreatic adenocarcinoma, insulinomas, gastrinomas, breast cancer, and sarcoma.

In some embodiments, the cancer is selected from neuroendocrine tumors, prostate cancer, renal cancer, bladder cancer, brain cancer, gastrointestinal cancer, medullar thyroid carcinomas, small or non-small cell lung cancer, stromal ovarian carcinomas, insulinomas, gastrinomas, breast cancer, and sarcoma.

In some embodiments, the cancer is selected from neuroendocrine tumors, prostate cancer, small cell lung cancer, breast cancer, and hepatocellular cancer.

In addition, to the treatment of cancer, the combined administration of PAH and a radiolabeled complex reduces the nephrotoxic side effects of the radiolabeled complex. Accordingly, the combination of PAH or a salt thereof, as described above, with a radiolabeled complex, as described above, may also be used (in addition to the treatment of cancer) to reduce nephrotoxic side effects of the radiolabeled complex.

As used herein, the term "treatment" or "treating" of a disease includes preventing, reducing, delaying or protecting against the disease (that is, causing the clinical symptoms not to develop or to develop in reduced or delayed form); inhibiting or reducing the disease (i.e., arresting, delaying, reducing or suppressing the development of clinical symptoms); and/or relieving the disease (i.e., causing the regression of clinical symptoms). As will be appreciated, it is not always possible to distinguish between "preventing" and "suppressing" a disease or disorder since the ultimate inductive event or events may be unknown or latent. Accordingly, the term "prophylaxis" will be understood to constitute a type of "treatment" that encompasses both "preventing" and "suppressing." The term "treatment" thus includes "prophylaxis". Accordingly, the term "treatment" includes prophylactic treatment (before onset of the disease) as well as therapeutic treatment (after onset of the disease).

The terms "therapy" and "therapeutic", as used herein, preferably mean to have at least some minimal physiological effect upon being administered to a living body. For example, a physiological effect upon administering a "therapeutic" anti-tumor compound may be the inhibition of tumor growth, or decrease in tumor size, or prevention reoccurrence of the tumor. Preferably, in the treatment of cancer or neoplastic disease, a compound which inhibits the growth of a tumor or decreased the size of the tumor or prevents the reoccurrence of the tumor would be considered therapeutically effective. The term "anti-tumor drug" therefore preferably means any therapeutic agent having therapeutic effect against a tumor, neoplastic disease or cancer.

The components of the combination of the present invention as described herein, i.e. (i) the radiolabeled complex, as described above, and (ii) PAH or a salt thereof, as described above, are administered as combination therapy. This means that, even if one component (the radiolabeled complex or PAH) is not administered, e.g., at the same day as the other component (the other of the radiolabeled complex or PAH), their treatment schedules are typically intertwined. This means that "a combination" in the context of the present invention does in particular not include the start of a therapy with one component (the radiolabeled complex or PAH) after the therapy with the other component

(the other of the radiolabeled complex or PAH) is finished. Thereby, a "finished" therapy means in particular that the active component does not exert its effects anymore - i.e. a "therapy" may in particular be finished several minutes, hours or days after the last administration of the active component, depending on how long the active component exerts its (anti-cancer/anti-tumor) effects. In more general, an "intertwined" treatment schedule of the radiolabeled complex and PAH - and, thus, a combination of the radiolabeled complex and PAH - means that

(i) not every administration of the radiolabeled complex (and therefore the complete therapy with the radiolabeled complex) is completed for more than one week (preferably for more than 3 days, more preferably for more than 2 days, even more preferably for more than a day) before the first administration of PAH or a salt thereof (and therefore the complete therapy with PAH) starts; or
(ii) not every administration of PAH or a salt thereof (and therefore the complete therapy with PAH) is completed for more than one week (preferably for more than 3 days, more preferably for more than 2 days, even more preferably for more than a day) before the first administration of the radiolabeled complex (and therefore the complete therapy with the radiolabeled complex) starts.

For example, in the combination of the radiolabeled complex as described herein and of PAH or a salt thereof, as described herein, one component (the radiolabeled complex or PAH) may be administered once a week and the other component (the other of the radiolabeled complex or PAH) may be administered once a month. To achieve in this example "a combination" in the sense of the present invention the monthly administered component is to be administered at least once in the same week, in which also the weekly administered other component is administered.
The combinations and pharmaceutical compositions as described herein may be administered only once (single administration). In particular, the radiolabeled complex as described herein may be administered only once (single administration). PAH, or the salt thereof, as described herein, may be administered once or twice.
In some embodiments, the combination or pharmaceutical composition is administered repeatedly, e.g. 2, 3, 4, 5, 6, 7, 8, 9 or 10 times, preferably not more than seven times, more preferably not more than five times, even more preferably not more than 3 times. In particular, the radiolabeled complex as described herein is preferably administered not more than seven times, more preferably not more than five times, even more preferably not more than 3 times. Usually, a single or very few administrations of the radionuclide complex are sufficient to exert its anti-tumor effects.
Preferably, the radiolabeled complex and/or the PAH (or the salt thereof) comprised in the combination according to the present invention may be administered at the same day. For example, if the radiolabeled complex and/or the PAH (or the salt thereof) comprised in the combination according to the present invention are administered repeatedly and at those days, at which the radiolabeled complex is administered, also the PAH (or the salt thereof) is administered.
In the combination of the radiolabeled complex, as described herein, and of the PAH (or the salt thereof), as described herein, the radiolabeled complex and the PAH (or the salt thereof) are preferably administered at about the same time.
"At about the same time", as used herein, refers in particular to simultaneous administration.
It also encompasses situations, where directly after administration of the radiolabeled complex the PAH (or the salt thereof) is administered or directly after administration of the PAH (or the salt thereof) the radiolabeled complex is administered. The skilled person understands that "directly after" includes the time necessary to prepare the second administration - in particular the time necessary for exposing and disinfecting the location for the second administration as well as appropriate preparation of the "administration device" (e.g., syringe, pump, etc.). Simultaneous administration also includes if the periods of administration of the radiolabeled complex and of the PAH (or the salt thereof) overlap or if, for example, one component (radiolabeled complex or PAH) is administered over a longer period of time, such as 30 min, 1 h, 2 h or even more, e.g. by infusion, and the other component (the other of the radiolabeled complex or PAH) is administered at some time during such a long period.
Preferably, the radiolabeled complex, as described herein, and the PAH (or the salt thereof), as described herein, are administered at the same day, more preferably at about the same time, and even more preferably simultaneously. This may be achieved by a single combined administration only. In some embodiments, PAH (or the salt thereof), as described herein, may be administered (without concomitant administration of the radiolabeled complex) before the combined administration of the radiolabeled complex, as described herein, and the PAH (or the salt thereof), as described herein (at about the same time, e.g. simultaneously). For example, PAH (without concomitant administration of the radiolabeled complex) may be administered no more than 120 min, preferably no more than 90 min, more preferably no more than 60 min, even more preferably no more than 30 min, still more preferably no more than 20 min, such as no more than 15 or 10 min, before the combined administration of the radiolabeled complex, as described herein, and the PAH (or the salt thereof), as described herein (at about the same time, e.g. simultaneously).
PAH or the salt thereof, preferably aminohippurate sodium, may be administered prior and during or prior and after administration of the radiolabeled complex. The pre-administration of PAH may be about 60 min, 30 min, 10 min or 5 min prior to the administration of the radiolabeled complex, preferably PAH is administered about 0.5-5h or 10-60

min prior to prior to the administration of the radiolabeled complex. In certain embodiments PAH may be administered prior to the administration of the radiolabeled complex and thereafter, e.g. 0.5-5h or 10-60 min thereafter, or prior, during and thereafter.

In some embodiments, pre-administration of PAH (or the salt thereof), preferably para-aminohippurate sodium, may be administered 5 to 30 min or 5 to 20 min or 5 to 15 min prior to the administration of the radiolabeled complex (in particular as continuous administration over 5 to 30 min or 5 to 20 min or 5 to 15 min) and continues for another 50 to 90 min or 40 to 60 min (e.g. upon the start of the administration of the radiolabeled complex). Thereby, the total continuous administration period of PAH (or a salt thereof) may be 60 to 90min or 60 to 80 min. The radiolabeled complex may be continuously administered for 10 to 25 or 10 to 20 min concomitant with the administration of PAH (or a salt thereof).

In particular, PAH (or a salt thereof) and the radiolabeled complex are administered systemically, preferably intravenously. Advantageously, the administrations may be performed by infusion, e.g. by means of a syringe or infusion pump and an infusion catheter. The administration of both components may be efficiently enabled by a three way stopcock or luer lock. The syringe or infusion pumps for administration of PAH (or a salt thereof) solution and the radiolabeled complex solution may thus be connected to distinct ports of the three way stopcock or luer lock.

The duration of the administration of PAH (or a salt thereof) and/or the radiolabeled complex may depend on the flow rate, the volume, and the concentration of PAH (or a salt thereof) and the radiolabeled complex in the administered solutions. The flow rate of PAH (or a salt thereof) may be chosen from 0.9 mL/min to about 2 mL/min. The flow rate of the radiolabeled complex may be chosen from 1.7 mL/min to about 2.5 mL/min. In some embodiments, the flow rate of both components may be kept constant (over the course of their continuous administration). In other embodiments, the flow rate of one or both components may be altered in the course of their continuous administration. In some embodiments, the flow rate of PAH (or a salt thereof) is altered by starting with a higher flow rate (priming dose), in particular during the pre-administration period, and by reducing the flow rate shortly before (e.g. 0.2 to 3 min) or essentially simultaneously with the onset of the administration of the radiolabeled complex. The flow rate of PAH may be reduced by 15 to 40% relative to its start value, e.g. to 0.9 to 1.3 mL/min. In some embodiments, the flow rate of PAH (or a salt thereof) is altered by starting with a higher flow rate (priming dose), in particular during the pre-administration period, and by reducing the flow rate shortly before (e.g. 0.2 to 3 min) or essentially simultaneously with the onset of the administration of the radiolabeled complex, while the flow rate of the radiolabeled complex is kept constant throughout its administration period. In some embodiments, the concentration of PAH (or a salt thereof) in the solution administered according to the treatment protocol as disclosed herein may be between 1 mg and 3 mg/20 mL or 1.5 to 2.5 mg/20 mL, e.g. it may be a 5 to 15% solution. The volume of the administered PAH (or a salt thereof) solution, in particular an aqueous solution, may be in the range of 75 to 130 mL or 90 to 120 mL.

In some embodiments, the patient's plasma concentration of PAH (or a salt thereof) in the course of the PAH (or a salt thereof) administration, in particular at the end of PAH (or a salt thereof) administration, achieves 500 to 900 mg/mL or 700 to 900 mg/mL.

Advantageously, fluid, in particular water intake by the subject to be treated, e.g. the cancer patient suffering from neuroendocrine tumors, prostate cancer, small cell lung cancer, breast cancer, or hepatocellular cancer, is ensured ahead of the start of the treatment protocol as disclosed herein. A preferably oral fluid intake of 0.5 to 1L within 30 to 60 min ahead of the treatment protocol is preferred.

It is understood that for medical purposes, "an effective amount" of the radiolabeled complex and of PAH (or a salt thereof) is administered. For example, if the radiolabeled complex and/or PAH is comprised in a (pharmaceutical) composition, the (pharmaceutical) composition usually comprises an effective amount of the radiolabeled complex. As used herein, "an effective amount" means an amount of the agent(s) that is sufficient to allow for significantly induction of a positive modification of the disease to be treated. At the same time, however, an "effective amount" may be small enough to avoid serious side-effects, that is to say to permit a sensible relationship between advantage and risk. An "effective amount" may vary depending on the particular condition to be treated and also with the age and physical condition of the patient to be treated, the severity of the condition, the duration of the treatment, the nature of the accompanying therapy, of the particular pharmaceutically acceptable excipient or carrier used, and similar factors. Accordingly, an "effective amount" may be readily determined in a specific situation by the physician. In general, effective doses may be determined by routine experiments, e.g. by using animal models. Such models include, without implying any limitation, rabbit, sheep, mouse, rat, dog and non-human primate models. Therapeutic efficacy and toxicity of radiolabeled complexes and PAH can be determined by standard pharmaceutical procedures in cell cultures or experimental animals, e.g., for determining the LD50 (the dose lethal to 50% of the population) and the ED50 (the dose therapeutically effective in 50% of the population).

The dose ratio between toxic and therapeutic effects is the therapeutic index and can be expressed as the ratio LD50/ED50. The data obtained from the cell culture assays and animal studies can be used in determining a dose range for use in humans. The dose of said conjugates lies preferably within a range of circulating concentrations that include the ED50 with little or no toxicity.

The dosage administered, as single or multiple doses, to an individual will thus vary depending upon a variety

of factors, including pharmacokinetic properties, subject conditions and characteristics (sex, age, body weight, health, size), extent of symptoms, concurrent treatments, frequency of treatment and the effect desired.

The radiolabeled complex comprised in the combination according to the present invention and the PAH (or a salt or thereof) comprised in the combination according to the present invention can be administered by various routes of administration, for example, systemically or locally (e.g. intratumorally). Systemic administration, in particular parental administration, is preferred. Non-limiting examples of preferred routes of administration include intravenous (i.v.), intravasal, subcutaneous, intramuscular and intradermal administration. In some embodiments, intravenous administration is preferred. Alternatively, administration may be accomplished locally, for instance at the site of affliction, such as intratumoral administration.

Preferably, the radiolabeled complex comprised in the combination according to the present invention and the PAH (or a salt thereof) comprised in the combination according to the present invention are administered systemically. In some embodiments, the radiolabeled complex is administered intratumorally and the PAH (or a salt thereof) is administered systemically.

Preferably, the radiolabeled complex comprised in the combination according to the present invention and the PAH (or a salt thereof) comprised in the combination according to the present invention are administered via the same route of administration, preferably via the same systemic route of administration, more preferably via the same parenteral route of administration, even more preferably intravenously.

In some embodiments, the combination according to the present invention comprising (a) the radiolabeled complex and (b) PAH (or a salt thereof), as described above, may be administered in combination with further, additional active compounds (e.g., in the context of tumor/cancer treatment). In other embodiments, the combination according to the present invention, comprising (a) the radiolabeled complex and (b) PAH (or a salt thereof), as described above, is not administered in combination with further, additional active compounds (e.g., in the context of tumor/cancer treatment). In other words, the inventive combination may also be useful as "stand-alone" therapy.

In general, each of the components (a) the radiolabeled complex and (b) PAH (or a salt thereof) of the combination may be comprised in the same composition or in separate compositions. Preferably, the components (a) the radiolabeled complex and (b) PAH (or a salt thereof) of the combination may be comprised in the same composition. Accordingly, each of the components (a) the radiolabeled complex and (b) PAH (or a salt thereof) of the combination may be comprised in a separate container (e.g., a syringe). Preferably, the components ((a) the radiolabeled complex and (b) PAH (or a salt thereof) of the combination may be comprised in the same container (e.g., a syringe).

Accordingly, the present invention also provides a combination of compositions, wherein a first composition comprises the radiolabeled complex as described above (but preferably not the PAH or a salt thereof as described above); and a second composition comprises the PAH (or a salt thereof) as described above (but preferably not the radiolabeled complex as described above), for use in the treatment of cancer.

Preferably, the components (a) the radiolabeled complex and (b) PAH (or a salt thereof) of the combination are comprised in the same composition. Accordingly, the present invention also provides a pharmaceutical composition comprising (a) the radiolabeled complex, as described herein, and (b) PAH (or a salt thereof), as described herein, for use in the treatment of cancer, whereby the combined administration of (a) and (b) increases the anti-tumor efficacy of (a); and wherein the pharmaceutical composition further comprises a pharmaceutically acceptable excipient, diluent or carrier.

Further details of such compositions are described below. In general, a (pharmaceutical) composition may comprise a pharmaceutically acceptable excipient, diluent or carrier as described below.

*Compositions*

The details outlined above including the details regarding the radiolabeled complex (in particular regarding the radionuclide, the targeting molecule and the chelating agent); PAH (or a salt thereof); the administration (in particular regarding schedule and routes of administration); the cancers to be treated; etc. apply accordingly to the pharmaceutical compositions of the invention.

For example, also in the pharmaceutical composition of the invention the targeting molecule is selected from peptides, peptidomimetics, antibody fragments, antibody mimetics and folate.

Preferably, the targeting molecule is selected from somatostatin analogues, PSMA-inhibitors, gastrin analogues, integrin binding molecules and folate, as described above. More preferably, the targeting molecule binds to PSMA or a somatostatin receptor, as described above. The targeting molecule may be selected from the group consisting of Tyr3-octeotride, Tyr3-octreotate, JR11, PSMA-11, Sargastrin, RGD and folate, preferably the targeting molecule is octreotide, more preferably Tyr3-octeotride, as described above.

For example, also in the pharmaceutical composition of the invention the chelating agent may be a macrocyclic chelator, preferably selected from the group consisting of DOTA, HBED-CC, NOTA, NODAGA, DOTAGA, DOTAM, TRAP, NOPO, PCTA, DFO, DTPA, DO3AP, DO3AP$^{PrA}$, DO3AP$^{ABn}$, and HYNIC or derivatives thereof, as described

above. More preferably, the chelating agent is DOTA, as described above. Even more preferably, the radiolabeled complex comprises or consists of (i) the radionuclide and (ii) DOTATOC or DOTATATE, as described above.

For example, also in the pharmaceutical composition of the invention the radionuclide may be selected from the group consisting of $^{94}Tc$, $^{99m}Tc$, $^{90}In$, $^{111}In$, $^{67}Ga$, $^{68}Ga$, $^{86}Y$, $^{90}Y$, $^{177}Lu$, $^{161}Tb$, $^{186}Re$, $^{188}Re$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{227}Th$, $^{153}Sm$, $^{166}Ho$, $^{166}Dy$, $^{18}F$ and $^{131}I$, as described above; preferably selected from the group consisting of $^{90}In$, $^{111}In$, $^{67}Ga$, $^{68}Ga$, $^{86}Y$, $^{90}Y$, $^{177}Lu$, $^{161}Tb$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{153}Sm$, $^{166}Ho$, $^{225}Ac$ and $^{166}Dy$; more preferably the radionuclide is selected from the group consisting of $^{177}Lu$, $^{68}Ga$ $^{111}In$, $^{90}Y$, $^{99m}Tc$, $^{225}Ac$ and $^{161}Tb$; even more preferably $^{177}Lu$, $^{225}Ac$ and $^{68}Ga$, or selected from a tri-valent radionuclide, preferably selected from the group consisting of $^{177}Lu$, $^{90}Y$, $^{67}Ga$, $^{68}Ga$, $^{111}In$, $^{225}Ac$, $^{161}Tb$, $^{44}Sc$ and $^{47}Sc$. Particularly preferably, the radionuclide is $^{177}Lu$ (Lutetium-177) , as described above.

For example, also in the pharmaceutical composition of the invention the radiolabeled complex is preferably selected from [$^{177}Lu$-DOTA°-Tyr3]-octreotide, $^{177}Lu$-DOTA-JA11, $^{177}Lu$-DOTA-RGD, $^{177}Lu$-DOTA-Sargastrin, and $^{177}Lu$-PSMA-I&T, as described above; more preferably the radiolabeled complex is $^{177}Lu$-DOTATOC, as described above.

For example, also the pharmaceutical composition of the invention may comprise the radiolabeled complex and PAH, as described above.

Preferably, the pharmaceutical composition of the invention may comprise the radiolabeled complex as described above and a salt of PAH, as described above; more preferably the pharmaceutical composition of the invention may comprise the radiolabeled complex as described above and sodium para-aminohippurate.

For example, also in the pharmaceutical composition of the invention PAH (or the salt thereof) is preferably administered in a concentration of 5 mg to 500 mg per kilogram of body weight, as described above.

For example, also in the pharmaceutical composition of the invention (a) the radiolabeled complex and (b) PAH or the salt thereof are present in a ratio from 1/240000 to 1/8000 (w/w), as described above.

In general, PAH or the pharmaceutically acceptable salt thereof, preferably aminohippurate sodium, may be present in the pharmaceutical composition in a larger quantity than the radiolabeled complex.

For instance, the radiolabeled complex and PAH or a pharmaceutically acceptable salt thereof, preferably aminohippurate sodium, are present in the pharmaceutical composition in a ratio of about 1/1.000.000 to 1/10 (w/w), preferably of about 1/500.000 to 1/100 (w/w), more preferably from about 1/250.000 to about 1/500 (w/w).

Preferably, the radiolabeled complex and PAH or a pharmaceutically acceptable salt thereof, preferably aminohippurate sodium, are present in the pharmaceutical composition in a ratio of about 1/250.000 to about 1/5.000 (w/w), for example in a ratio of about 1/250.000, 1/200.000, 1/150.000, 1/100.000, or 1/50.000 to about 1/5.000 (w/w), more preferably in a ratio of about 1/240.000 to about 1/8.000 (w/w), for example in a ratio of about 1/240.000, 1/230.000, 1/220.000, 1/210.000, 1/200.000, 1/190.000, 1/180.000, 1/170.000, 1/160.000, 1/150.000, 1/140.000, 1/130.000, 1/120.000, 1/110.000, 1/100.000, 1/90.000, 1/80.000, 1/70.000, 1/60.000, 1/50.000, 1/40.000, 1/30.000, 1/20.000, 1/19.000, 1/18.000, 1/17.000, 1/16.000, 1/15.000, 1/14.000, 1/13.000, 1/12.000, 1/11.000, 1/10.000, 1/9.000, or 1/8.000 (w/w). More preferably, the radiolabeled complex and PAH or a pharmaceutically acceptable salt thereof, preferably aminohippurate sodium, are present in the pharmaceutical composition in a ratio of about 1/100.000 to about 1/10.000 (w/w), for example in a ratio of about 1/100.000, 1/95.000, 1/90,000, 1/85.000, 1/80.000, 1/75.000, 1/70.000, 1/65.000, 1/60.000 1/55.000, 1/50.000, 1/45.000, 1/40.000, 1/35.000, 1/30.000, 1/25.000, 1/20.000, 1/15.000, 1/10.000 (w/w). It is also preferred that the radiolabeled complex and PAH or a pharmaceutically acceptable salt thereof, preferably aminohippurate sodium, are present in the pharmaceutical composition in a ratio of about 1/50.000 to about 1/40.000 (w/w), for example in a ratio of about 1/50.000, 1/49.000, 1/48.000, 1/47.000, 1/46.000, 1/45.000, 1/44.000, 1/43.000, 1/42.000, 1/41.000, 1/40.000 (w/w).

## Formulations, carriers and excipients

The (pharmaceutical composition) is preferably a liquid or semi-liquid composition, which is more preferably a liquid or semi-liquid composition, which is more preferably an aqueous solution, which may be buffered and/or exhibit isotonic properties.

As described above, the (pharmaceutical) composition comprises a pharmaceutically acceptable excipient, diluent or carrier. The term "pharmaceutically acceptable", as used herein, refers to a compound or agent that is compatible with the components of the pharmaceutical composition, in particular the active (anti-cancer) compounds, and does not interfere with and/or substantially reduce its therapeutic activities. Pharmaceutically acceptable carriers preferably have sufficiently high purity and sufficiently low toxicity to make them suitable for administration to a subject to be treated.

Pharmaceutically acceptable excipients can exhibit different functional roles and include, without limitation, diluents, fillers, bulking agents, carriers, disintegrants, binders, lubricants, glidants, coatings, solvents and co-solvents, buffering agents, preservatives, adjuvants, antioxidants, wetting agents, anti-foaming agents, thickening agents, sweetening agents, flavouring agents and humectants.

Suitable pharmaceutically acceptable excipients are typically chosen based on the formulation of the pharmaceutical composition.

For pharmaceutical compositions in liquid form, useful pharmaceutically acceptable excipients in general include solvents, diluents or carriers such as (pyrogen-free) water, (isotonic) saline solutions such phosphate or citrate buffered saline, fixed oils, vegetable oils, such as, for example, groundnut oil, cottonseed oil, sesame oil, olive oil, corn oil, ethanol, polyols (for example, glycerol, propylene glycol, polyetheylene glycol, and the like); lecithin; surfactants; preservatives such as benzyl alcohol, parabens, chlorobutanol, phenol, ascorbic acid, thimerosal, and the like; isotonic agents such as sugars, polyalcohols such as manitol, sorbitol, or sodium chloride; aluminum monostearate or gelatin; antioxidants such as ascorbic acid or sodium bisulfite; chelating agents such as ethylenediaminetetraacetic acid (EDTA); buffers such as acetates, citrates or phosphates and agents for the adjustment of tonicity such as sodium chloride or dextrose. pH can be adjusted with acids or bases, such as hydrochloric acid or sodium hydroxide. Buffers may be hypertonic, isotonic or hypotonic with reference to the specific reference medium, i.e. the buffer may have a higher, identical or lower salt content with reference to the specific reference medium, wherein preferably such concentrations of the aforementioned salts may be used, which do not lead to damage of cells due to osmosis or other concentration effects. Reference media are e.g. liquids occurring in *in vivo* methods, such as blood, lymph, cytosolic liquids, or other body liquids, or e.g. liquids, which may be used as reference media in *in vitro* methods, such as common buffers or liquids. Such common buffers or liquids are known to a skilled person.

Liquid pharmaceutical compositions administered via injection and in particular via intravasal, more preferably intravenous (i.v.) injection should preferably be sterile and stable under the conditions of manufacture and storage. Such compositions are typically formulated as parenterally acceptable aqueous solutions that are pyrogen-free, have suitable pH, are isotonic and maintain stability of the active ingredient(s).

For liquid pharmaceutical compositions, suitable pharmaceutically acceptable excipients and carriers include water, typically pyrogen-free water; isotonic saline or buffered (aqueous) solutions, e.g. phosphate, citrate etc. buffered solutions. Particularly for injection of the (pharmaceutical) compositions, water or preferably a buffer, more preferably an aqueous buffer, may be used, which may contain a sodium salt, e.g. at least 50 mM of a sodium salt, a calcium salt, e.g. at least 0,01 mM of a calcium salt, and optionally a potassium salt, e.g. at least 3 mM of a potassium salt.

The sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, NaI, NaBr, $Na_2CO_3$, $NaHCO_3$, $Na_2SO_4$, examples of the optional potassium salts include e.g. KCl, KI, KBr, $K_2CO_3$, $KHCO_3$, $K_2SO_4$, and examples of calcium salts include e.g. $CaCl_2$, $CaI_2$, $CaBr_2$, $CaCO_3$, $CaSO_4$, $Ca(OH)_2$. Furthermore, organic anions of the aforementioned cations may be contained in the buffer.

Buffers suitable for injection purposes as defined above, may contain salts selected from sodium chloride (NaCl), calcium chloride ($CaCl_2$) and optionally potassium chloride (KCl), wherein further anions may be present additional to the chlorides. $CaCl_2$ can also be replaced by another salt like KCl. Typically, the salts in the injection buffer are present in a concentration of at least 50 mM sodium chloride (NaCl), at least 3 mM potassium chloride (KCl) and at least 0,01 mM calcium chloride ($CaCl_2$). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects.

For pharmaceutical compositions in (semi-)solid form, suitable pharmaceutically acceptable excipients and carriers include binders such as microcrystalline cellulose, gum tragacanth or gelatine; starch or lactose; sugars, such as, for example, lactose, glucose and sucrose; starches, such as, for example, corn starch or potato starch; cellulose and its derivatives, such as, for example, sodium carboxymethylcellulose, ethylcellulose, cellulose acetate; disintegrants such as alginic acid; lubricants such as magnesium stearate; glidants such as stearic acid, magnesium stearate; calcium sulphate, colloidal silicon dioxide and the like; sweetening agents such as sucrose or saccharin; and/or flavoring agents such as peppermint, methyl salicylate, or orange flavoring.

Generally, pharmaceutical compositions for topical administration can be formulated as creams, ointments, gels, pastes or powders. Pharmaceutical compositions for oral administration can be formulated as tablets, capsules, liquids, powders or in a sustained release format. However, according to preferred embodiments, the pharmaceutical composition is administered parenterally, in particular via intravenous or intratumoral injection, and is accordingly formulated in liquid or lyophilized form for parenteral administration. Parenteral formulations may be stored in vials, IV bags, ampoules, cartridges, or prefilled syringes and can be administered as injections, inhalants, or aerosols, with injections being preferred.

The pharmaceutical composition may be provided in lyophilized form. Lyophilized pharmaceutical compositions are preferably reconstituted in a suitable buffer, advantageously based on an aqueous carrier, prior to administration.

The pharmaceutical composition is preferably an aqueous solution, in particular a radiopharmaceutical aqueous solution. As used herein, an "aqueous solution" is usually a solution of one or more solute(s) in water. The pharmaceutical

composition may be for intravenous (IV) use/application/administration. The pharmaceutical composition is typically stable, concentrated, and ready-to-use.

The pharmaceutical compositions are also provided for use in the preparation of a medicament for the treatment of cancer.

*Kits*

In a further aspect, the present invention also provides a kit (of parts) comprising

(a) a radiolabeled complex comprising (i) a radionuclide and (ii) a targeting molecule linked to a chelating agent, wherein the targeting molecule is selected from peptides, peptidomimetics, antibody fragments, antibody mimetics and folate;
(b) para-aminohippuric acid (PAH); or a sall thereof;
(c) an instruction leaflet, package insert or label with directions for administering (a) and/or (b) for use in the treatment of cancer, whereby the combined administration of (a) and (b) increases the anti-tumor efficacy of (a).

The details outlined above including the details regarding the radiolabeled complex (in particular regarding the radionuclide, the targeting molecule and the chelating agent); PAH (or a salt thereof); the administration (in particular regarding schedule and routes of administration); the cancers to be treated; etc. apply accordingly to the kits of the invention.

For example, also in the kit of the invention the targeting molecule is selected from peptides, peptidomimetics, antibody fragments, antibody mimetics and folate.

Preferably, the targeting molecule is selected from somatostatin analogues, PSMA-inhibitors, gastrin analogues, integrin binding molecules and folate, as described above. More preferably, the targeting molecule binds to PSMA or a somatostatin receptor, as described above. The targeting molecule may be selected from the group consisting of Tyr3-octeotride, Tyr3-octreotate, JR11, PSMA-11, Sargastrin, RGD and folate, preferably the targeting molecule is octreotide, more preferably Tyr3-octeotride, as described above.

For example, also in the kit of the invention the chelating agent may be a macrocyclic chelator, preferably selected from the group consisting of DOTA, HBED-CC, NOTA, NODAGA, DOTAGA, DOTAM, TRAP, NOPO, PCTA, DFO, DTPA, DO3AP, DO3AP$^{PrA}$, DO3AP$^{ABn}$, and HYNIC or derivatives thereof, as described above. More preferably, the chelating agent is DOTA, as described above. Even more preferably, the radiolabeled complex comprises or consists of (i) the radionuclide and (ii) DOTATOC or DOTATATE, as described above.

For example, also in the kit of the invention the radionuclide may be selected from the group consisting of $^{94}$Tc, $^{99m}$Tc, $^{90}$In, $^{171}$In, $^{67}$Ga, $^{68}$Ga, $^{86}$Y, $^{90}$Y, $^{177}$Lu, $^{161}$Tb, $^{186}$Re, $^{188}$Re, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{227}$Th, $^{153}$Sm, $^{166}$Ho, $^{166}$Dy, $^{18}$F and $^{131}$I, as described above; preferably selected from the group consisting of $^{90}$In, $^{111}$In, $^{67}$Ga, $^{68}$Ga, $^{86}$Y, $^{90}$Y, $^{177}$Lu, $^{161}$Tb, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{153}$Sm, $^{166}$Ho, $^{225}$Ac and $^{166}$Dy; more preferably the radionuclide is selected from the group consisting of $^{177}$Lu, $^{68}$Ga $^{111}$In, $^{90}$Y, $^{99m}$Tc, $^{225}$Ac and $^{161}$Tb; even more preferably $^{177}$Lu, $^{225}$Ac and $^{68}$Ga, or selected from a tri-valent radionuclide, preferably selected from the group consisting of $^{177}$Lu, $^{90}$Y, $^{67}$Ga, $^{68}$Ga, $^{111}$In, $^{225}$Ac, $^{161}$Tb, $^{44}$Sc and $^{47}$Sc. Particularly preferably, the radionuclide is $^{177}$Lu (Lutetium-177) , as described above.

For example, also in the kit of the invention the radiolabeled complex is preferably selected from [$^{177}$Lu-DOTA°-Tyr3]-octreotide, $^{177}$Lu-DOTA-JA11, $^{177}$Lu-DOTA-RGD, $^{177}$Lu-DOTA-Sargastrin, and $^{177}$Lu-PSMA I&T, as described above; more preferably the radiolabeled complex is $^{177}$Lu-DOTATOC, as described above.

For example, also the kit of the invention may comprise the radiolabeled complex and PAH, as described above.

Preferably, the kit of the invention may comprise the radiolabeled complex as described above and a salt of PAH, as described above; more preferably the kit of the invention may comprise the radiolabeled complex as described above and sodium para-aminohippurate.

In general, each of the components (a) the radiolabeled complex and (b) PAH (or a salt thereof) of the kit may be comprised in a separate container (e.g., a syringe). Preferably, the components ((a) the radiolabeled complex and (b) PAH (or a salt thereof) of the kit may be comprised in the same container (e.g., a syringe). Accordingly, each of the components (a) the radiolabeled complex and (b) PAH (or a salt thereof) of the kit may be comprised in separate or in the same (pharmaceutical) composition, as described above.

For example, the kit may comprise para-aminohippuric acid (PAH) or a pharmaceutically acceptable salt thereof in one part of the kit, and the radiolabeled complex, as specified above, in another part of the kit. The kit may also comprise (in a distinct part) a solution for e.g. diluting PAH (or a pharmaceutically acceptable salt thereof) and/or the radiolabeled complex. The solution may be isotonic or hypertonic, it may be buffered, e.g. an optionally buffered aqueous solution, e.g. an aqueous NaCl solution or water for injection (WFI).

Optionally, the kit may comprise at least one further agent, e.g. amino acids, such as lysine and arginine and

mixtures thereof, gelatine, Amifostine, albumin-derived peptides, PSMA-binding molecules, such as PMPA, vitamins, radionuclides, antimicrobial agents, solubilizing agents or the like.

The kit may be a kit of two or more parts comprising any of the components exemplified above in suitable containers. For example, each container may be in the form of vials, bottles, squeeze bottles, jars, sealed sleeves, envelopes or pouches, tubes or blister packages or any other suitable form, provided the container preferably prevents premature mixing of components. Each of the different components may be provided separately, or some of the different components may be provided together (i.e. in the same container), as described above.

A container may also be a compartment or a chamber within a vial, a tube, a jar, or an envelope, or a sleeve, or a blister package or a bottle, provided that the contents of one compartment are not able to associate physically with the contents of another compartment prior to their deliberate mixing by a pharmacist or physician.

The kit comprises an instruction leaflet, package insert or label with directions to administer (a) the radiolabeled complex and/or (b) PAH or the salt eir as described above.

**BRIEF DESCRIPTION OF THE FIGURES**

In the following a brief description of the appended figures will be given. The figures are intended to illustrate the present invention in more detail. However, they are not intended to limit the subject matter of the invention in any way.

Figure 1 shows for Example 1 the experimental design. Mice were assigned to three distinct groups receiving either vehicle only (NaCl 0.9%; Group 0), $^{177}$Lu-DOTATOC alone (no PAH; Group 1.1), or $^{177}$Lu-DOTATOC combined with PAH (Group 1.2) on day 7 after inoculation with tumor cells.

Figure 2 shows for Example 1 the tumor growth of the three experimental groups Group 0 ("control"), Group 1.1 ("NaCl") and Group 1.2 ("PAH") over the 57-day observation period after treatment.

Figure 3 shows for Example 1 the body weight ratios of the three experimental groups Group 0 ("Control group"), Group 1.1 ("177Lu-DOTATOC/NaCl 0.9%") and Group 1.2 ("177Lu-DOTATOC/PA"); (weight at different time points compared to initial body weight at day 0).

Figure 4 shows for Example 1 the results of a linear regression analysis performed for the tumor volume plotted against the actual tumor weight for each individual tumor (n= 36; 2 tumors/mouse).

Figure 5 shows for Example 1 the survival rates of the distinct experimental groups Group 0 ("Control"), Group 1.1 ("177Lu-DOTATOC+NaCl") and Group 1.2 ("177Lu-DOTATOC+PAH").

Figure 6 shows for Example 2 the tumor growth of the three experimental groups Group 1 ("177Lu-DOTATOC/NaCl 0.9%"), Group 2 ("1 77Lu-DOTATOC/PA") and Group 3 ("Control group - NaCl 0.9%") over the 46-day observation period after treatment.

Figure 7 shows for Example 2 the body weight ratios of the three experimental groups Group 1 ("177Lu-DOTATOC/NaCl 0.9%"), Group 2 ("177Lu-DOTATOC/PA") and Group 3 ("Control group - NaCl 0.9%"); (weight at different time points compared to initial body weight at day 0).

Figure 8 shows for Example 2 the results of a linear regression analysis performed for the tumor volume plotted against the actual tumor weight for each individual tumor.

Figure 9 shows for Example 2 the survival rates of the distinct experimental groups Group 1 ("177Lu-DOTATOC/NaCl 0.9%"), Group 2 ("177Lu-DOTATOC/PA") and Group 3 ("Control group - NaCl 0.9%").

EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only.

**Example 1: Combination of PAH and a radiolabeled complex in a mouse tumor model**

To investigate the effects of a combination of para-aminohippuric acid (PAH) and a radiolabeled complex, an *in vivo tumor* model, namely, the AR42J pancreatic tumor-bearing nude mouse model, was used. As radiolabeled compound, a $^{177}$Lu-labelled compound, $^{177}$Lu-DOTATOC, was used.

Briefly, CD1 nude mice (Crl: CD1-*Foxn1$^{nu}$;* 8 weeks at beginning of experiment) were used.

For the preparation of the AR42J xenograft tumor, AR42J cell line (rat *pancreatic* acinar cells) were cultured at 37°C and 5% $CO_2$ in Ham's F-12K medium (Gibco, Ref 21127022) supplemented with inactivated 20% FBS (Sigma Aldrich, Ref F7524). Subcultivation was performed every seven days by trypsinization and aliquoted in new flasks. A suspension of AR42J cells at 50 x $10^6$ cells/mL was prepared in Ham's F-12K medium (without fetal bovine serum). Mice were inoculated subcutaneously in the both right and left flanks with 5 x $10^6$ cells in 100 μL of Ham's F-12K medium. After inoculation, tumor growth was controlled by visual observation and palpation until the dosing day (7 days post-inoculation). Only animals showing apparent nodules were included in the experiment.

For the experiment, 18 tumor-bearing mice were assigned to three distinct experimental groups as shown in Table 1 below:

Table 1.

| Group | 1.1 | 1.2 | 0 |
|---|---|---|---|
| **$^{177}$Lu-DOTATOC** | + | + | - |
| **PAH** | - | + | - |
| **Number of mice** | 6 | 6 | 6 |

The experimental design is shown in Fig. 1. At day 7 after inoculation with the tumor cells mice received a single intravenous injection as indicated in Table 1, i.e. either with vehicle (0.9% NaCl; Group 0), or with 40 MBq $^{177}$Lu-DOTATOC, alone (in 0.9% NaCl; Group 1.1) or in combination with PAH (in 20% PAH solution (Merck Sharp & Dohme Corp); Group 1.2) 10 min prior to dosing of $^{177}$Lu-DOTATOC, mice of groups 1.1 and 1.2 were injected intraperitoneally with 50 μL saline or PAH, respectively.

Body weight and tumor volume were evaluated every two to three days for a period of 57 days after treatment (or until the tumor size limit was reached). The tumor volume was determined by measuring the length, the width and the depth of the tumor with a digital caliper. Tumor volume was calculated by using the following formula :

$$\text{Tumor volume} = (\text{length x width x depth}) \times 0.5$$

The mice were monitored until the individual tumor size reached 1500 $mm^3$. When this experimental endpoint was reached or after a 57 days period following treatment, mice were euthanized. Mean tumor volume and body weight of AR42J xenograft CD1 nude mice were calculated for each treatment group throughout the duration of the study.

The mean values of the tumor volume and the body weight of mice with respective standard errors (SD) for each treatment group throughout the duration of the study are shown in Table 2 and Table 3 below:

Table 2: Tumor volume (mean ± SD $mm^3$)

| Days post- dosing | Group 0 | Group 1.1 | Group 1.2 |
|---|---|---|---|
| **0** | 0 ± 0(n=6) | 0 ± 0(n=6) | 0 ± 0(n=6) |
| **1** | 0 ± 0 | 0 ± 0 ± | 0 ± 0 |
| **4** | 1 ± 2 | 0 ± 0 | 0 ± 0 |
| **6** | 2±3 | 0 ± 0 | 0 ± 0 |
| **8** | 22 ± 42 | 0 ± 0 | 0 ± 0 |
| **11** | 60 ± 72 | 0 ± 0 | 0 ± 0 |
| **13** | 128 ± 153 | 0 ± 0 | 0 ± 0 |
| **15** | 242 ± 244 | 0 ± 0 | 0 ± 0 |
| **18** | 443 ± 418 | 2 ± 6 | 0 ± 0 |

(continued)

| Days post- dosing | Group 0 | Group 1.1 | Group 1.2 |
|---|---|---|---|
| **20** | 629 ± 544 | 10 ± 22 | 13 ± 31 |
| **22** | 661 ± 551 (n=5) | 33 ± 47 | 20 ± 33 |
| **25** | 673 ± 621 (n=3) | 122 ± 120 | 76 ± 96 |
| **27** | 535 ± 415(n=2) | 175 ± 182 | 106 ± 140 |
| **29** | 724± 624 | 251 ± 233 | 159 ± 206 |
| **32** | 777± 805(n=1) | 516 ± 347 | 322 ± 360 |
| **34** | 847 ± 829 | 709 ± 466 | 466 ± 527 |
| **36** | | 786 ± 338(n=4) | 457 ± 547(n=5) |
| **39** | | 1192 ± 486 | 500 ± 705(n=4) |
| **41** | | 1265 ± 199(n=2) | 138 ± 157(n=3) |
| **43** | | | 215 ± 241 |
| **46** | | | 395 ± 411 |
| **48** | | | 534 ± 516 |
| **50** | | | 690 ± 655 |
| **53** | | | 673 ± 22(n=1) |
| **55** | | | 820 ± 101 |
| | | | 1010 ± 98 |

Table 3: Body weight (mean ± SD %ID/g)

| Days post- dosing | Group 0 | Group 1.1 | Group 1.2 |
|---|---|---|---|
| **0** | 27.3 ± 2.2 (n=6) | 25.0 ± 0.8 (n=6) | 27.0 ± 3.1 (n=6) |
| **1** | 27.1 ± 2.1 | 24.7 ± 1.1 | 26.7 ± 3.3 |
| **4** | 27.4 ± 1.7 | 25.4 ± 0.8 | 26.7 ± 3.3 |
| **6** | 27.9 ± 1.8 | 25.9 ± 0.9 | 27.8 ± 3.3 |
| **8** | 27.8 ± 1.9 | 25.8 ± 0.8 | 28.0 ± 3.2 |
| **11** | 28.4 ± 1.9 | 25.9 ± 0.9 | 28.1 ± 3.3 |
| **13** | 28.8 ± 1.9 | 25.9 ± 1.1 | 28.4 ± 3.1 |
| **15** | 29.0 ± 1.9 | 25.9 ± 1.1 | 28.2 ± 3.1 |
| **18** | 30.2 ± 2.4 | 26.0 ± 0.9 | 28.6 ± 2.9 |
| **20** | 30.4 ± 2.7 | 26.1 ± 0.9 | 28.6 ± 2.8 |
| **22** | 31.3 + 2.4 (n=5) | 26.3 ± 0.9 | 28.7 ± 2.7 |
| **25** | 31.7 ± 2.4 (n=3) | 26.2 ± 1.0 | 28.6 ± 2.5 |
| **27** | 31.5 ± 0.7 (n=2) | 27.0 ± 1.2 | 29.5 ± 2.5 |
| **29** | 31.9 ± 1.4 | 26.9 ± 0.9 | 29.5 ± 2.3 |
| **32** | 31.7 ± 2.2 | 28.1 ± 1.3 | 30.5 ± 2.1 |
| **34** | 32.8 ± 2.1 (n=1) | 29.0 ± 1.4 | 31.1 ± 2.1 |
| **36** | | 28.4 ± 0.8 (n=4) | 31.6 ± 2.0 (n=5) |
| **39** | | 29.5 ± 0.9 | 31.9 ± 3.0 (n=4) |

(continued)

| Days post- dosing | Group 0 | Group 1.1 | Group 1.2 |
|---|---|---|---|
| **41** | | 30.6 + 0.4 (n=2) | 31.4 ± 3.0 (n=3) |
| **43** | | | 31.1 ± 2.8 |
| **46** | | | 31.8 ± 3.2 |
| **48** | | | 33.0 ± 3.5 |
| **50** | | | 33.3 ± 4.1 |
| **53** | | | 29.6 (n=1) |
| **55** | | | 30.6 |
| **57** | | | 30.6 |

The tumor growth curve and the body weight ratios of mice (weight at different time points compared to initial body weight at D0) are shown in Figures 2 and 3, respectively. As mice exhibiting a tumor size >1500 $mm^3$ were taken out of the experiment, the average tumor volume shown in the curves was affected accordingly.

Figure 2 shows a significant tumor growth delay of 10 days when mice were injected with $^{177}$Lu-DOTATOC compared to the control group. The treatment with $^{177}$Lu-DOTATOC suppressed tumor growth successfully for 20 days, revealing a substantial tumor growth inhibition. Furthermore, an even slower resumption of tumor growth was observed after treatment with $^{177}$Lu-DOTATOC combined with PAH as compared to treatment with $^{177}$Lu-DOTATOC alone.

Figure 3 reveals that the average weight of AR42J xenograft CD1 nude mice treated with radiolabeled DOTATOC increased similarly to that the untreated tumor-bearing control mice. Thus, mice treated with radiolabeled DOTATOC as well as untreated control mice maintained a healthy physical appearance throughout the study period.

The tumor volume was then plotted against actual tumor weight for each individual tumor (n= 36; 2 tumors/mouse) and a linear regression analysis was performed. The results are shown in Figure 4. In Figure 4, the linear regression analysis demonstrated that the correlation between tumor volume and weight was highly significant ($r^2$ = 0.9598). Thus, these data established that the formula for estimating tumor volume reflected the actual tumor size accurately.

Figure 5 shows the survival curves for each treatment group. For each group, the median survival time (MST) to reach the maximum tumor size of 1500 $mm^3$ was calculated. The median survival time was 23 days for the control group (Group 0) versus 39 days for Group 1.1 ($^{177}$Lu-DOTATOC alone) and 44.5 days Group 1.2 ($^{177}$Lu-DOTATOC in combination with PAH).

In summary, while administration of the radiolabeled complex alone decreased/delayed tumor growth and increased the survival times of tumor-bearing mice, combined administration of the radiolabeled complex and PAH even further decreased/delayed tumor growth and increased the survival times of tumor-bearing mice.

## Example 2: Combination of PAH and a radiolabeled complex in a mouse tumor model

This study was performed to confirm the effects observed in Example 1 for a combination of para-aminohippuric acid (PAH) and a radiolabeled complex, in an *in vivo* tumor model, namely, the AR42J pancreatic tumor-bearing nude mouse model. Similarly as in Example 1, $^{177}$Lu-DOTATOC was used as radiolabeled compound.

Briefly, SWISS nude mice (Crl: NU(Ico)-*Foxn1$^{nu}$*; 8 weeks at beginning of experiment) were used. For the preparation of the AR42J xenograft tumor, AR42J cell line (rat *pancreatic* acinar cells) were cultured at 37°C and 5% $CO_2$ in Ham's F-12K medium (Gibco, Ref 21127022) supplemented with inactivated 20% FBS (Sigma Aldrich, Ref F7524). Subcultivation was performed every seven days by trypsinization and aliquoted in new flasks. A suspension of AR42J cells at 50 x $10^6$ cells/mL was prepared in Ham's F-12K medium (without fetal bovine serum). Mice were inoculated subcutaneously in the right flank with 5 x $10^6$ cells in 100 μL of Ham's F-12K medium. After inoculation, tumor growth was controlled by visual observation and palpation until the dosing day (7 days post-inoculation). Only animals showing apparent nodules were included in the experiment.

For the experiment, 36 tumor-bearing mice were assigned to three distinct experimental groups as shown in Table 4 below:

Table 4.

| Group | 1 | 2 | 3 |
|---|---|---|---|
| $^{177}$Lu-DOTATOC | + | + | - |
| PAH | - | + | - |
| Number of mice | 12 | 12 | 12 |

The experimental design essentially corresponds to Example 1, as shown in Fig. 1. At day 7 after inoculation with tumor cells, mice received a single intravenous injection as indicated in Table 1, i.e. either with vehicle (0.9% NaCl; Group 3), or with 41-42 MBq $^{177}$Lu-DOTATOC, alone (in 0.9% NaCl; Group 1) or in combination with PAH (in 20% PAH solution (Merck Sharp & Dohme Corp); Group 2). 10 min prior to dosing of $^{177}$Lu-DOTATOC, mice of groups 1 and 2 were injected intraperitoneally with 50 $\mu$L saline or PAH, respectively.

Body weight and tumor volume were evaluated every two to three days for a period of 46 days after treatment (or until the tumor size limit was reached). The tumor volume was determined by measuring the length, the width and the depth of the tumor with a digital caliper. Tumor volume was calculated by using the following formula :

$$\text{Tumor volume} = (\text{length x width x depth}) \text{ x } 0.5$$

The mice were monitored until the individual tumor size reached 1500 mm$^3$. When this experimental endpoint was reached or after a 46 days period following treatment, mice were euthanized. Mean tumor volume and body weight of AR42J xenograft SWISS mice were calculated for each treatment group throughout the duration of the study.

The mean values of the tumor volume and the body weight of mice with respective standard errors (SD) for each treatment group throughout the duration of the study are shown in Table 5 and Table 6 below:

Table 5: Tumor volume (mean ± SD mm$^3$)

| Days post- dosing | Group 3 | Group 1 | Group 2 |
|---|---|---|---|
| 1 | 0 ± 0 (n=12) | 0 ± 0(n=12) | 0 ± 0 (n=12) |
| 4 | 8 ± 27 | 0 ± 0 | 0 ± 0 |
| 6 | 40 ± 75 | 0 ± 0 | 0 ± 0 |
| 8 | 90 ± 94 | 0 ± 0 | 0 ± 0 |
| 11 | 371 ± 288 | 0 ± 0 | 0 ± 0 |
| 13 | 659 ± 445 | 0 ± 0 | 0 ± 0 |
| 15 | 892 ± 409 (n=11) | 0 ± 0 | 0 ± 0 |
| 18 | 1216 ± 483 (n=9) | 29 ± 37 | 38 ± 58 |
| 20 | 951 ± 380 (n=4) | 59 ± 48 | 79 ± 93 |
| 22 | 1181 ± 570 | 147 ± 130 | 169 ± 194 |
| 25 | 965 ± 87 (n=2) | 410 ± 255 | 342 ± 356 |
| 27 | 1137 ± 104 | 674 ± 362 | 604 ± 548 |
| 29 | 1362 ± 246 | 1033 ± 461 (n=11) | 793 ± 605 (n=11) |
| 32 | 1238 (n=1) | 1285 ± 458 (n=7) | 714 ± 504 (n=8) |
| 34 | 1663 | 1396 ± 408 (n=4) | 920 ± 620 (n=7) |
| 36 | | 1425 ± 419 (n=2) | 779 ± 516 (n=5) |
| 39 | | 1567(n=1) | 822 ± 311 (n=4) |
| 41 | | | 1186 ± 468 |
| 43 | | | 1346 ± 222 (n=2) |
| 46 | | | 1892 (n=1) |

Table 6: Body weight (mean ± SD g)

| Days post- dosing | Group 3 | Group 1 | Group 2 |
|---|---|---|---|
| **1** | 21.0± 1.2 (n=12) | 24.3 ± 1.7 (n=12) | 23.8 ± 2,1 (n=12) |
| **4** | 22.1± 1.1 | 24.3 ± 1.6 | 23.5 ± 2,2 |
| **6** | 22.1± 1.1 | 24.5 ± 1.9 | 23.8 ± 2,2 |
| **8** | 22.6± 0.9 | 24.9 ± 1.9 | 24.4 ± 2,2 |
| **11** | 23.1± 1.0 | 24.8 ± 1.9 | 24.4 ± 2,1 |
| **13** | 23.6± 1.1 | 24.8 ± 1.9 | 24.6 ± 1,9 |
| **15** | 23.9± 1.4 (n=11) | 24.7 ± 1.9 | 24.6 ± 2,0 |
| **18** | 24.9± 1.6 (n=9) | 24.9 ± 1.9 | 24.8 ± 2,1 |
| **20** | 25.4± 1.9 (n=4) | 25.2 ± 1.8 | 24.8 ± 2,2 |
| **22** | 26.0± 1.7 | 25.6 ± 1.8 | 25.2 ± 2,5 |
| **25** | 25.7± 1.3 (n=2) | 26.2 ± 1.9 | 25.6 ± 2,6 |
| **27** | 26.3± 1.1 | 26.8 ± 1.8 | 26.4 ± 2,6 |
| **29** | 27.0± 1.2 | 26.9 ± 1.9 (n=11) | 26.6 ± 2,7 (n=11) |
| **32** | 28.6 (n=1) | 27.5 ± 2.0 (n=7) | 26.7 ± 2.5 (n=8) |
| **34** | 29.5 | 28.1 ± 1.1 (n=4) | 27.4 ± 2.8 (n=7) |
| **36** | | 28.3 ± 1.1 (n=2) | 26.6 ± 1,5 (n=5) |
| **39** | | 29.6 (n=1) | 27.6 ± 2.0 (n=4) |
| **41** | | | 28.4 ± 2,1 |
| **43** | | | 27.7 ± 2,7 (n=2) |
| **46** | | | 27.5 (n=1) |

The tumor growth curve and the body weight ratios of mice (weight at different time points compared to initial body weight at D0) are shown in Figures 6 and 7, respectively. As mice exhibiting a tumor size >1500 $mm^3$ were taken out of the experiment, the average tumor volume shown in the curves was affected accordingly.

Figure 6 shows a significant tumor growth delay of about 14 days when mice were injected with $^{177}$Lu-DOTATOC compared to the control group. The treatment with $^{177}$Lu-DOTATOC suppressed tumor growth successfully for 15 days, revealing a substantial tumor growth inhibition. An even slower tumor growth was observed after treatment with $^{177}$Lu-DOTATOC combined with PAH as compared to treatment with $^{177}$Lu-DOTATOC alone.

Figure 7 reveals that no significant weight loss was observed regardless of the treatment administered to the mice suggesting a low radiotoxicity elicited by radiolabeled compound. However, the average weight of AR42J xenograft mice treated with the radiolabeled compound increased slightly more slowly than the tumor-bearing control mice. Independent of the combination with PAH, mice treated $^{177}$Lu-DOTATOC showed the same weight gain throughout the experiment. Thus, mice treated with radiolabeled DOTATOC as well as untreated control mice maintained a healthy physical appearance throughout the study period.

In Figure 8, the linear regression analysis demonstrated that the correlation between tumor volume and weight was highly significant ($r^2$ = 0.9101). Thus, these data established that the formula for estimating tumor volume reflected the actual tumor size accurately.

Figure 9 shows the survival curves for each treatment group. As expected, the control group had to be euthanized earlier due the faster tumor growth than the two groups treated with radiolabeled DOTATOC. Similarly as in Example 1, survival time was prolonged when $^{177}$Lu-DOTATOC was combined with PAH as compared to $^{177}$Lu-DOTATOC alone.

Accordingly Example 2 confirmed the results of Example 1, namely, that combined administration of the radiolabeled complex and PAH even further decreased/delayed tumor growth and increased the survival times of tumor-bearing mice.

## Claims

1. A combination of

   (a) a radiolabeled complex comprising (i) a radionuclide and (ii) a targeting molecule linked to a chelating agent, wherein the targeting molecule is selected from peptides, peptidomimetics, antibody fragments, antibody mimetics and folate; and
   (b) para-aminohippuric acid (PAH), or a salt thereof; for use in the treatment of cancer, wherein the combined administration of (a) and (b) increases the anti-tumor efficacy of (a).

2. The combination for use according to claim 1, wherein the targeting molecule is selected from somatostatin analogues, PSMA-inhibitors, gastrin analogues, integrin binding molecules and folate.

3. The combination for use according to any one of the previous claims, wherein the targeting molecule binds to PSMA or a somatostatin receptor (SSTR).

4. The combination for use according to any one of the previous claims, wherein the targeting molecule is selected from the group consisting of Tyr3-octeotride, Tyr3-octreotate, JR11, PSMA-11, Sargastrin, RGD and folate, wherein the targeting molecule is preferably octreotide, more preferably Tyr3-octeotride.

5. The combination for use according to any one of the previous claims, wherein the chelating agent is a macrocyclic chelator, preferably selected from the group consisting of DOTA, HBED-CC, NOTA, NODAGA, DOTAGA, DOTAM, TRAP, NOPO, PCTA, DFO, DTPA, DO3AP, DO3AP$^{PrA}$, DO3AP$^{ABn}$, and HYNIC ,
   wherein the chelating agent is preferably DOTA.

6. The combination for use according to any one of the previous claims, wherein the radiolabeled complex comprises or consists of (i) the radionuclide and (ii) DOTATOC or DOTATATE.

7. The combination for use according to any one of the previous claims, wherein the radionuclide is selected from the group consisting of $^{94}$Tc, $^{99m}$Tc, $^{90}$In, $^{111}$In, $^{67}$Ga, $^{68}$Ga, $^{86}$Y, $^{90}$Y, $^{177}$Lu, $^{161}$Tb, $^{186}$Re, $^{188}$Re, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{227}$Th, $^{153}$Sm, $^{166}$Ho, $^{166}$Dy, $^{18}$F and $^{131}$I, preferably selected from the group consisting of $^{90}$In, $^{111}$In, $^{67}$Ga, $^{68}$Ga, $^{86}$Y, $^{90}$Y, $^{177}$Lu, $^{161}$Tb, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{153}$Sm, $^{166}$Ho, $^{225}$Ac and $^{166}$Dy,

   wherein the radionuclide is preferably selected from the group consisting of $^{177}$Lu, $^{68}$Ga $^{111}$In, $^{90}$Y, $^{99m}$Tc, $^{225}$Ac and $^{161}$Tb, more preferably $^{177}$Lu, $^{225}$Ac and $^{68}$Ga, or selected from a tri-valent radionuclide, preferably selected from the group consisting of $^{177}$Lu, $^{90}$Y, $^{67}$Ga, $^{68}$Ga, $^{111}$In, $^{225}$Ac, $^{161}$Tb, $^{44}$Sc and $^{47}$Sc,
   wherein the radionuclide is more preferably $^{177}$Lu (Lutetium-177).

8. The combination for use according to any one of the previous claims, wherein the radiolabeled complex is selected from [$^{177}$Lu-DOTA°-Tyr3]-octreotide, $^{177}$Lu-DOTA-JR11, $^{177}$Lu-DOTA-RGD, $^{177}$Lu-DOTA-Sargastrin, and $^{177}$Lu-PSMA I&T,
   wherein the radiolabeled complex is preferably $^{177}$Lu-DOTATOC.

9. The combination for use according to any one of claims 1 - 8, wherein the combination comprises

   (a) the radiolabeled complex; and
   (b) para-aminohippuric acid (PAH) or a salt of para-aminohippuric acid (PAH), wherein the salt of PAH is sodium para-aminohippurate.

10. The combination for use according to any one of the previous claims, wherein PAH or the salt thereof is administered in a concentration of 5 mg to 500 mg per kilogram of body weight,
    and/or
    wherein (a) the radiolabeled complex and (b) PAH or the salt thereof are administered in a ratio from 1/240000 to 1/8000 (w/w).

11. The combination for use according to any one of the previous claims, wherein (a) the radiolabeled complex and (b) PAH or the salt thereof are administered on the same day,

and/or
wherein (a) the radiolabeled complex and (b) PAH or the salt thereof are administered at the same time.

**12.** The combination for use according to any one of the previous claims, wherein (a) the radiolabeled complex and (b) PAH or the salt thereof are administered via the same route of administration.

**13.** The combination for use according to any one of the previous claims, wherein (a) the radiolabeled complex and (b) PAH or the salt thereof are administered systemically.

**14.** The combination for use according to any one of the previous claims, wherein (a) the radiolabeled complex and (b) PAH or the salt thereof are administered in the same composition.

**15.** The combination for use according to any one of the previous claims, wherein the cancer is selected from neuroendocrine tumors, prostate cancer, pancreatic cancer, renal cancer, bladder cancer, medullar thyroid carcinomas, small cell lung cancers, stromal ovarian carcinomas, ductal pancreatic adenocarcinoma, insulinomas, gastrinomas, and breast cancer.

**16.** The combination for use according to claim 15, wherein the cancer is selected from neuroendocrine tumors, prostate cancer, small cell lung cancer, and breast cancer, in particular neuroendocrine tumors.

**17.** The combination for use according to any one of the previous claims, wherein the subject in need of the treatment is a human cancer patient.

**18.** A kit comprising

(a) a radiolabeled complex comprising (i) a radionuclide and (ii) a targeting molecule linked to a chelating agent, wherein the targeting molecule is selected from peptides, peptidomimetics, antibody fragments, antibody mimetics and folate;
(b) para-aminohippuric acid (PAH), or a salt thereof; and
(c) an instruction leaflet, package insert or label with directions for administering (a)
and/or (b)

for use in the treatment of cancer, whereby the combined administration of (a) and (b) increases the anti-tumor efficacy of (a).

**19.** The kit for use according to claim 18, wherein the radiolabelled complex is as defined in any one of claims 2-8,
and/or
wherein the para-aminohippuric acid (PAH) or the salt thereof is as defined by claim 9.

**20.** A pharmaceutical composition comprising

(a) a radiolabeled complex comprising (i) a radionuclide and (ii) a targeting molecule linked to a chelating agent, wherein the targeting molecule is selected from peptides, peptidomimetics, antibody fragments, antibody mimetics and folate; and
(b) para-aminohippuric acid (PAH) or a salt thereof; for use in the treatment of cancer, whereby the combined administration of (a) and (b) increases the anti-tumor efficacy of (a); and wherein the pharmaceutical composition further comprises a pharmaceutically acceptable excipient, diluent or carrier.

**21.** The pharmaceutical composition for use according to claim 20, wherein the composition is an aqueous solution.

**22.** The pharmaceutical composition for use according to claim 20 or 21, wherein the

radiolabelled complex is as defined in any one of claims 2-8 and/or
wherein the para-aminohippuric acid (PAH) or the salt thereof is as defined by claim 9.

**Patentansprüche**

1. Kombination aus

   (a) einem radioaktiv markierten Komplex, umfassend (i) ein Radionuklid und (ii) ein Targeting-Molekül, das mit einem Chelatbildner verbunden ist, wobei das Targeting-Molekül aus Peptiden, Peptidomimetika, Antikörperfragmenten; Antikörpermimetika und Folat ausgewählt ist; und
   (b) para-Aminohippursäure (PAH) oder einem Salz davon;

   zur Verwendung bei der Behandlung von Krebs, wobei die kombinierte Verabreichung von (a) und (b) die Anti-Tumor-Wirksamkeit von (a) erhöht.

2. Kombination zur Verwendung gemäß Anspruch 1, wobei das Targeting-Molekül ausgewählt ist aus Somatostatin-Analoga, PSMA-Inhibitoren, Gastrin-Analoga, Integrin-bindenden Molekülen und Folat.

3. Kombination zur Verwendung gemäß irgendeinem der vorhergehenden Ansprüche, wobei das Targeting-Molekül an PSMA oder einen Somatostatinrezeptor (SSTR) bindet.

4. Kombination zur Verwendung gemäß irgendeinem der vorhergehenden Ansprüche,

   wobei das Targeting-Molekül ausgewählt ist aus der Gruppe bestehend aus Tyr3-Octreotid, Tyr3-Octreotat, JR11, PSMA-11, Sargastrin, RGD und Folat,
   wobei das Targeting-Molekül bevorzugt Octreotid, bevorzugter Tyr3-Octreotid ist.

5. Kombination zur Verwendung gemäß irgendeinem der vorhergehenden Ansprüche,

   wobei der Chelatbildner ein makrozyklischer Chelator ist, vorzugsweise ausgewählt aus der Gruppe bestehend aus DOTA, HBED-CC, NOTA, NODAGA, DOTAGA, DOTAM, TRAP, NOPO, PCTA, DFO, DTPA, DO3AP, $DO3AP^{PrA}$, $DO3AP^{ABn}$ und HYNIC,
   wobei der Chelatbildner bevorzugt DOTA ist.

6. Kombination zur Verwendung gemäß irgendeinem der vorhergehenden Ansprüche, wobei der radioaktiv markierte Komplex (i) das Radionuklid und (ii) DOTATOC oder DOTATATE umfasst oder daraus besteht.

7. Kombination zur Verwendung gemäß irgendeinem der vorhergehenden Ansprüche,

   wobei das Radionuklid ausgewählt ist aus der Gruppe bestehend aus $^{94}Tc$, $^{99m}Tc$, $^{90}In$, $^{111}In$, $^{67}Ga$, $^{68}Ga$, $^{86}Y$, $^{90}Y$, $^{177}Lu$, $^{161}Tb$, $^{186}Re$, $^{188}Re$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{227}Th$, $^{153}Sm$, $^{166}Ho$, $^{166}Dy$, $^{18}F$ und $^{131}I$, vorzugsweise ausgewählt aus der Gruppe bestehend aus $^{90}In$, $^{111}In$, $^{67}Ga$, $^{68}Ga$, $^{86}Y$, $^{90}Y$, $^{177}Lu$, $^{161}Tb$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{153}Sm$, $^{166}Ho$, $^{225}Ac$ und $^{166}Dy$,
   wobei das Radionuklid bevorzugt ausgewählt ist aus der Gruppe bestehend aus $^{177}Lu$, $^{68}Ga$ $^{111}In$, $^{90}Y$, $^{99m}Tc$, $^{225}Ac$ und $^{161}Tb$, bevorzugter $^{177}Lu$, $^{225}Ac$ und $^{68}Ga$, oder ausgewählt ist aus einem dreiwertigen Radionuklid, bevorzugt ausgewählt aus der Gruppe bestehend aus $^{177}Lu$, $^{90}Y$, $^{67}Ga$, $^{68}Ga$, $^{111}In$, $^{225}Ac$, $^{161}Tb$, $^{44}Sc$ und $^{47}Sc$,
   wobei das Radionuklid bevorzugter $^{177}Lu$ (Lutetium-1 77) ist.

8. Kombination zur Verwendung gemäß irgendeinem der vorhergehenden Ansprüche, wobei der radioaktiv markierte Komplex ausgewählt ist aus [$^{177}Lu$-DOTA°-Tyr3]-Octreotid, $^{177}Lu$-DOTA-JR11, $^{177}Lu$-DOTA-RGD, $^{177}Lu$-DOTA-Sargastrin und $^{177}Lu$-PSMA I&T, wobei der radioaktiv markierte Komplex vorzugsweise $^{177}Lu$-DOTATOC ist.

9. Kombination zur Verwendung gemäß irgendeinem der Ansprüche 1 bis 8, wobei die Kombination umfasst

   (a) den radioaktiv markierten Komplex; und
   (b) para-Aminohippursäure (PAH) oder ein Salz der para-Aminohippursäure (PAH),

   wobei das Salz der PAH Natrium-para-Aminohippurat ist.

10. Kombination zur Verwendung gemäß irgendeinem der vorhergehenden Ansprüche,

wobei PAH oder das Salz davon in einer Konzentration von 5 mg bis 500 mg pro Kilogramm Körpergewicht verabreicht wird,
und/oder
wobei (a) der radioaktiv markierte Komplex und (b) PAH oder das Salz davon in einem Verhältnis von 1/240000 bis 1/8000 (w/w) verabreicht werden.

**11.** Kombination zur Verwendung gemäß irgendeinem der vorhergehenden Ansprüche,

wobei (a) der radioaktiv markierte Komplex und (b) PAH oder das Salz davon am selben Tag verabreicht werden, und/oder
wobei (a) der radioaktiv markierte Komplex und (b) PAH oder das Salz davon zur gleichen Zeit verabreicht werden.

**12.** Kombination zur Verwendung gemäß irgendeinem der vorhergehenden Ansprüche, wobei (a) der radioaktiv markierte Komplex und (b) PAH oder das Salz davon über denselben Verabreichungsweg verabreicht werden.

**13.** Kombination zur Verwendung gemäß irgendeinem der vorhergehenden Ansprüche, wobei (a) der radioaktiv markierte Komplex und (b) PAH oder das Salz davon systemisch verabreicht werden.

**14.** Kombination zur Verwendung gemäß irgendeinem der vorhergehenden Ansprüche, wobei (a) der radioaktiv markierte Komplex und (b) PAH oder das Salz davon in derselben Zusammensetzung verabreicht werden.

**15.** Kombination zur Verwendung gemäß irgendeinem der vorhergehenden Ansprüche, wobei der Krebs ausgewählt ist aus neuroendokrinen Tumoren, Prostatakrebs, Pankreaskrebs, Nierenkrebs, Blasenkrebs, medullären Schilddrüsenkarzinomen, kleinzelligen Lungenkarzinomen, stromalen Ovarialkarzinomen, duktalen Pankreasadenokarzinomen, Insulinomen, Gastrinomen und Brustkrebs.

**16.** Kombination zur Verwendung gemäß Anspruch 15, wobei der Krebs ausgewählt ist aus neuroendokrinen Tumoren, Prostatakrebs, kleinzelligem Lungenkrebs und Brustkrebs, insbesondere neuroendokrinen Tumoren.

**17.** Kombination zur Verwendung gemäß irgendeinem der vorhergehenden Ansprüche, wobei das Subjekt, das die Behandlung benötigt, ein menschlicher Krebspatient ist.

**18.** Kit, umfassend

(a) einen radioaktiv markierten Komplex, umfassend (i) ein Radionuklid und (ii) ein Targeting-Molekül, das an einen Chelatbildner gebunden ist, wobei das Targeting-Molekül aus Peptiden, Peptidomimetika, Antikörperfragmenten, Antikörpermimetika und Folat ausgewählt ist;
(b) para-Aminohippursäure (PAH) oder ein Salz davon; und
(c) eine Gebrauchsanweisung, einen Beipackzettel oder ein Etikett mit Anweisungen zur Verabreichung von (a) und/oder (b)

zur Verwendung bei der Behandlung von Krebs, wobei die kombinierte Verabreichung von (a) und (b) die Anti-Tumor-Wirksamkeit von (a) erhöht.

**19.** Kit zur Verwendung gemäß Anspruch 18, wobei der radioaktiv markierte Komplex wie in einem der Ansprüche 2 bis 8 definiert ist, und/oder
wobei die para-Aminohippursäure (PAH) oder das Salz davon wie in Anspruch 9 definiert ist.

**20.** Pharmazeutische Zusammensetzung, umfassend

(a) einen radioaktiv markierten Komplex, umfassend (i) ein Radionuklid und (ii) ein Targeting-Molekül, das an einen Chelatbildner gebunden ist; wobei das Targeting-Molekül aus Peptiden, Peptidomimetika, Antikörperfragmenten, Antikörpermimetika und Folat ausgewählt ist; und
(b) para-Aminohippursäure (PAH) oder ein Salz davon;

zur Verwendung bei der Behandlung von Krebs, wobei die kombinierte Verabreichung von (a) und (b) die Anti-Tumor-Wirksamkeit von (a) erhöht; und wobei die pharmazeutische Zusammensetzung außerdem einen pharmazeutisch akzeptablen Hilfsstoff, ein Verdünnungsmittel oder einen Träger umfasst.

**21.** Pharmazeutische Zusammensetzung zur Verwendung gemäß Anspruch 20, wobei die Zusammensetzung eine wässrige Lösung ist.

**22.** Pharmazeutische Zusammensetzung zur Verwendung gemäß Anspruch 20 oder 21, wobei der radiomarkierte Komplex wie in einem der Ansprüche 2 bis 8 definiert ist, und/oder
wobei die para-Aminohippursäure (PAH) oder das Salz davon wie in Anspruch 9 definiert ist.

**Revendications**

**1.** Combinaison de

(a) un complexe radiomarqué comprenant (i) un radionucléide et (ii) une molécule de ciblage liée à un agent de chélation, dans laquelle la molécule de ciblage est choisie parmi des peptides, des peptidomimétiques, des fragments d'anticorps, des mimétiques d'anticorps et du folate ; et
(b) l'acide para-aminohippurique (PAH) ou un de ses sels ;

pour son utilisation dans le traitement du cancer, dans laquelle l'administration combinée de (a) et (b) augmente l'efficacité antitumorale de (a).

**2.** Combinaison pour son utilisation selon la revendication 1, dans laquelle la molécule de ciblage est choisie parmi des analogues de la somatostatine, des inhibiteurs du PSMA, des analogues de la gastrine, des molécules de liaison à des intégrines et le folate.

**3.** Combinaison pour son utilisation selon l'une quelconque des revendications précédentes, dans laquelle la molécule de ciblage se lie au PSMA ou à un récepteur de la somatostatine (SSTR).

**4.** Combinaison pour son utilisation selon l'une quelconque des revendications précédentes, dans laquelle la molécule de ciblage est choisie parmi le groupe constitué par le Tyr3-octréotide, le Tyr3-octréotate, JR11, PSMA-11, la sargastrine, le RGD et le folate,
dans laquelle la molécule de ciblage représente de préférence l'octréotide, de manière plus préférée le Tyr3-octréotide.

**5.** Combinaison pour son utilisation selon l'une quelconque des revendications précédentes, dans laquelle l'agent de chélation est un chélateur macrocyclique, de préférence choisi parmi le groupe constitué par DOTA, HBED-CC, NOTA, NODAGA, DOTAGA, DOTAM, TRAP, NOPO, PCTA, DFO, DTPA, DO3AP, $DO3AP^{PrA}$, $DO3AP^{ABn}$, et HYNIC, dans laquelle l'agent de chélation représente de préférence DOTA.

**6.** Combinaison pour son utilisation selon l'une quelconque des revendications précédentes, dans laquelle le complexe radiomarqué comprend ou est constitué par (i) le radionucléide et (ii) DOTATOC ou DOTATATE.

**7.** Combinaison pour son utilisation selon l'une quelconque des revendications précédentes, dans laquelle le radionucléide est choisi parmi le groupe constitué par $^{94}Tc$, $^{99m}Tc$, $^{90}In$, $^{111}In$, $^{67}Ga$, $^{68}Ga$, $^{86}Y$, $^{90}Y$, $^{177}Lu$, $^{161}Tb$, $^{186}Re$, $^{188}Re$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{227}Th$, $^{153}Sm$, $^{166}Ho$, $^{166}Dy$, $^{18}F$ et $^{131}I$, de préférence choisi parmi le groupe constitué par $^{90}In$, $^{111}In$, 67Ga, $^{68}Ga$, $^{86}Y$, $^{90}Y$, $^{177}Lu$, $^{161}Tb$, $^{64}Cu$, $^{67}Cu$, $^{55}Co$, $^{57}Co$, $^{43}Sc$, $^{44}Sc$, $^{47}Sc$, $^{225}Ac$, $^{213}Bi$, $^{212}Bi$, $^{212}Pb$, $^{153}Sm$, $^{166}Ho$, $^{225}Ac$ et $^{166}Dy$,

dans laquelle le radionucléide est de préférence choisi parmi le groupe constitué par $^{177}Lu$, $^{68}Ga$, $^{111}In$, $^{90}Y$, $^{99m}Tc$, $^{225}Ac$ et $^{161}Tb$, de manière plus préférée par $^{177}Lu$, $^{225}Ac$ et $^{68}Ga$, ou est choisi parmi un radionucléide trivalent, de préférence choisi parmi le groupe constitué par $^{177}Lu$, $^{90}Y$, $^{67}Ga$, $^{68}Ga$, $^{111}In$, $^{225}Ac$, $^{161}Tb$ , $^{44}Sc$ et $^{47}Sc$,
dans laquelle le radionucléide représente de manière plus préférée $^{177}Lu$ (lutécium-177).

**8.** Combinaison pour son utilisation selon l'une quelconque des revendications précédentes, dans laquelle le complexe radiomarqué est choisi parmi le [$^{177}Lu$-DOTA$^{0}$-Tyr3]-octréptide, le $^{177}Lu$-DQTA-JR11, le $^{177}Lu$-DOTA-RGD, le $^{177}Lu$-DOTA-sargastrine, et le $^{177}Lu$-PSMA I&T.

**9.** Combinaison pour son utilisation selon l'une quelconque des revendications 1 à 8, dans laquelle la combinaison comprend

(a) le complexe radiomarqué ; et
(b) l'acide para-aminohippurique (PAH) ou un sel de l'acide para-aminohippurique (PAH),

dans laquelle le sel du PAH est le para-aminohippurate de sodium.

**10.** Combinaison pour son utilisation selon l'une quelconque des revendications précédentes, dans laquelle le PAH ou son sel est administré en une concentration de 5 mg à 500 mg par kilogramme de poids corporel,
et/ou
dans laquelle (a) le complexe radiomarqué et (b) le PAH ou son sel sont administrés dans un rapport de 1/240000 à 1/8000 (en poids/poids).

**11.** Combinaison pour son utilisation selon l'une quelconque des revendications précédentes, dans laquelle (a) le complexe radiomarqué et (b) le PAH ou son sel sont administrés le même jour,
et/ou
dans laquelle (a) le complexe radiomarqué et (b) le PAH ou son sel sont administrés en même temps.

**12.** Combinaison pour son utilisation selon l'une quelconque des revendications précédentes, dans laquelle (a) le complexe radiomarqué et (b) le PAH ou son sel sont administrés en empruntant la même voie d'administration.

**13.** Combinaison pour son utilisation selon l'une quelconque des revendications précédentes, dans laquelle (a) le complexe radiomarqué et (b) le PAH ou son sel sont administrés de manière systémique.

**14.** Combinaison pour son utilisation selon l'une quelconque des revendications précédentes, dans laquelle (a) le complexe radiomarqué et (b) le PAH ou son sel sont administrés dans la même composition.

**15.** Combinaison pour son utilisation selon l'une quelconque des revendications précédentes, dans laquelle le cancer est choisi parmi des tumeurs neuroendocrines, le cancer de la prostate, le cancer du pancréas, le cancer du rein, le cancer de la vessie, des cancers médullaires de la thyroïde, des cancers du poumon à petites cellules, des carcinomes des cellules stromales de l'ovaire, l'adénocarcinome ductal pancréatique, des insulinomes, des gastrinomes, et le cancer du sein.

**16.** Combinaison pour son utilisation selon la revendication 15, dans laquelle le cancer est choisi parmi des tumeurs neuroendocrines, le cancer de la prostate, le cancer du poumon à petites cellules, et le cancer du sein, en particulier concerne des tumeurs neuroendocrines.

**17.** Combinaison pour son utilisation selon l'une quelconque des revendications précédentes, dans laquelle le sujet nécessitant le traitement est un patient cancéreux humain.

**18.** Kit comprenant

(a) un complexe radiomarqué comprenant (i) un radionucléide et (ii) une molécule de ciblage liée à un agent de chélation, dans lequel la molécule de ciblage est choisie parmi des peptides, des peptidomimétiques, des fragments d'anticorps, des mimétiques d'anticorps et du folate ;
(b) l'acide para-aminohippurique (PAH) ou un de ses sels ; et
(c) une notice de mode d'emploi, un encart informatif ou une étiquette qui reprend des indications pour l'administration de (a) et/ou de (b), pour son utilisation dans le traitement du cancer, dans lequel l'administration combinée de (a) et de (b) augmente l'efficacité antitumorale de (a).

**19.** Kit pour son utilisation selon la revendication 18, dans lequel le complexe radiomarqué est tel que défini dans l'une quelconque des revendications 2 à 8,
et/ou
dans lequel l'acide para-aminohippurique (PAH) ou son sel est tel que défini par la revendication 9.

**20.** Composition pharmaceutique comprenant

(a) un complexe radiomarqué comprenant (i) un radionucléide et (ii) une molécule de ciblage liée à un agent de chélation, dans laquelle la molécule de ciblage est choisie parmi des peptides, des peptidomimétiques, des fragments d'anticorps, des mimétiques d'anticorps et du folate ; et
(b) l'acide para-aminohippurique (PAH) ou un de ses sels ; pour son utilisation dans le traitement du cancer, dans laquelle l'administration combinée de (a) et de (b) augmente l'efficacité antitumorale de (a) ;

et dans laquelle la composition pharmaceutique comprend en outre un excipient, un diluant ou un support pharmaceutiquement acceptable.

**21.** Composition pharmaceutique pour son utilisation selon la revendication 20, dans laquelle la composition est une solution aqueuse.

**22.** Composition pharmaceutique pour son utilisation selon la revendication 20 ou 21, dans laquelle le complexe radiomarqué est tel que défini dans l'une quelconque des revendications 2 à 8,
et/ou
dans laquelle l'acide para-aminohippurique (PAH) ou son sel est tel que défini par la revendication 9.

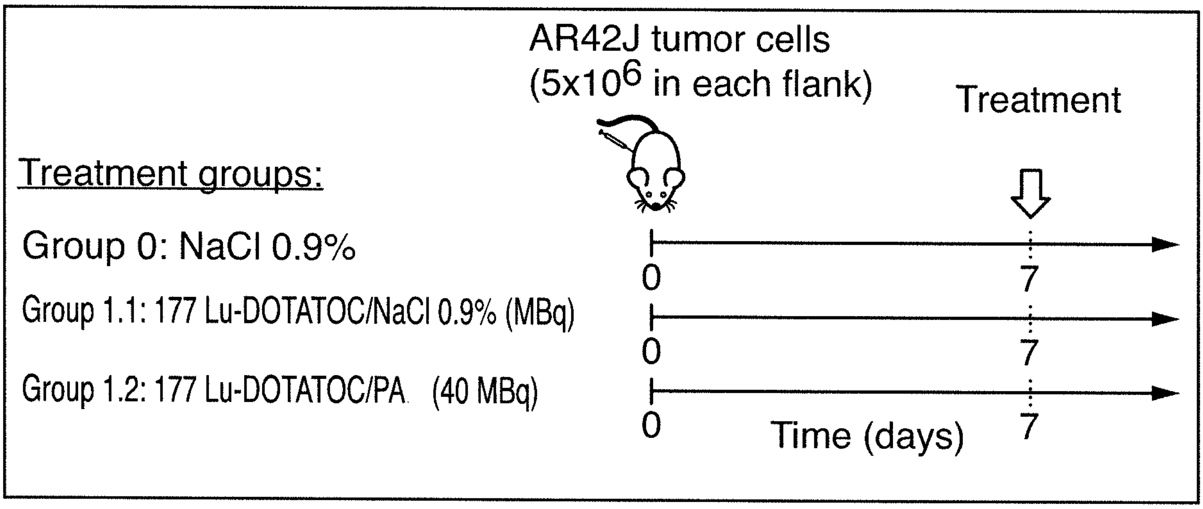
AR42J tumor cells
(5x10^6 in each flank)
Treatment
Treatment groups:
Group 0: NaCl 0.9%
Group 1.1: 177 Lu-DOTATOC/NaCl 0.9% (MBq)
Group 1.2: 177 Lu-DOTATOC/PA (40 MBq)
0
7
0
7
0
Time (days)
7

Figure 1

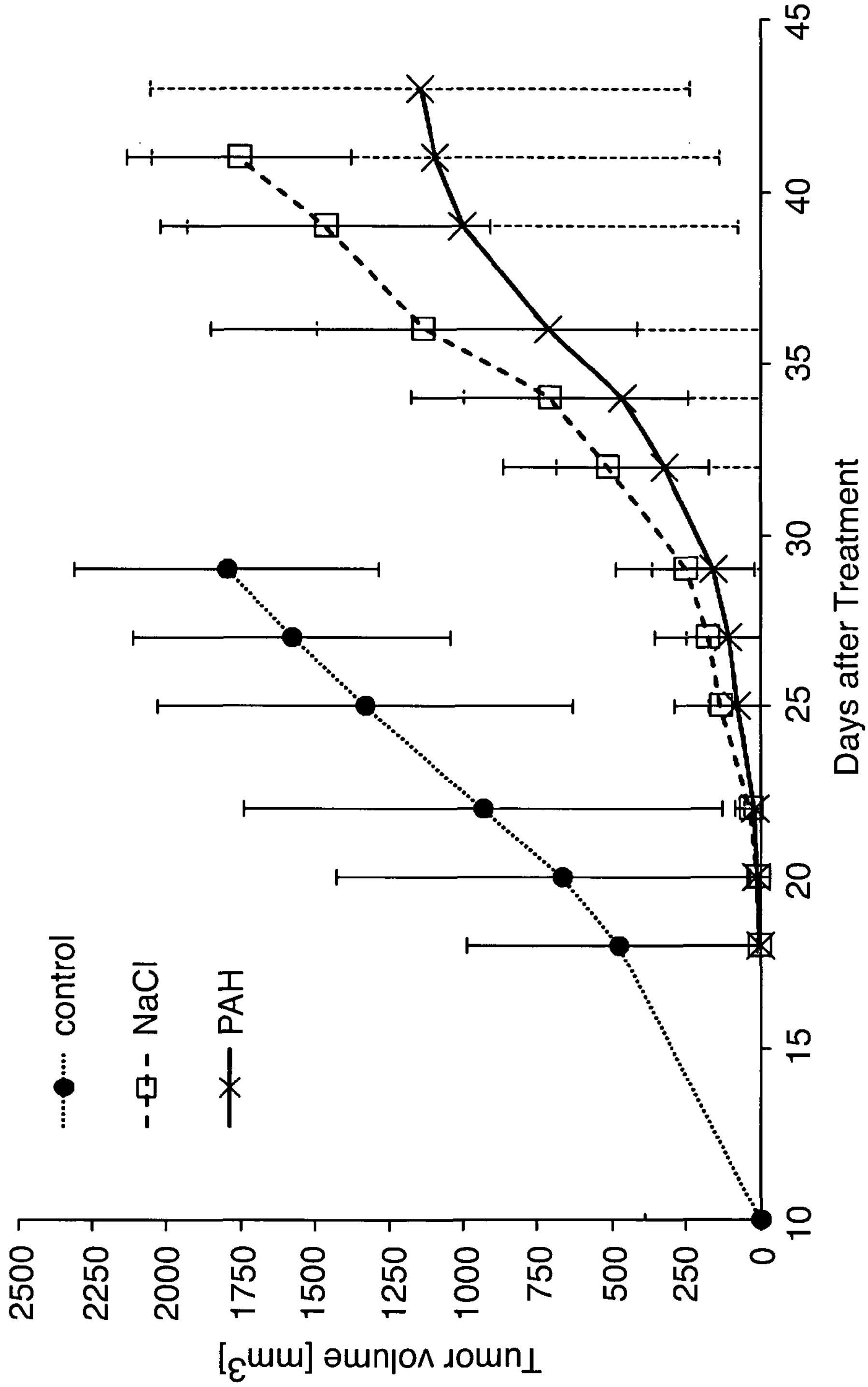
control
NaCl
PAH
Tumor volume [mm3]
2500
2250
2000
1750
1500
1250
1000
750
500
250
0
10
15
20
25
30
35
40
45
Days after Treatment

Figure 2

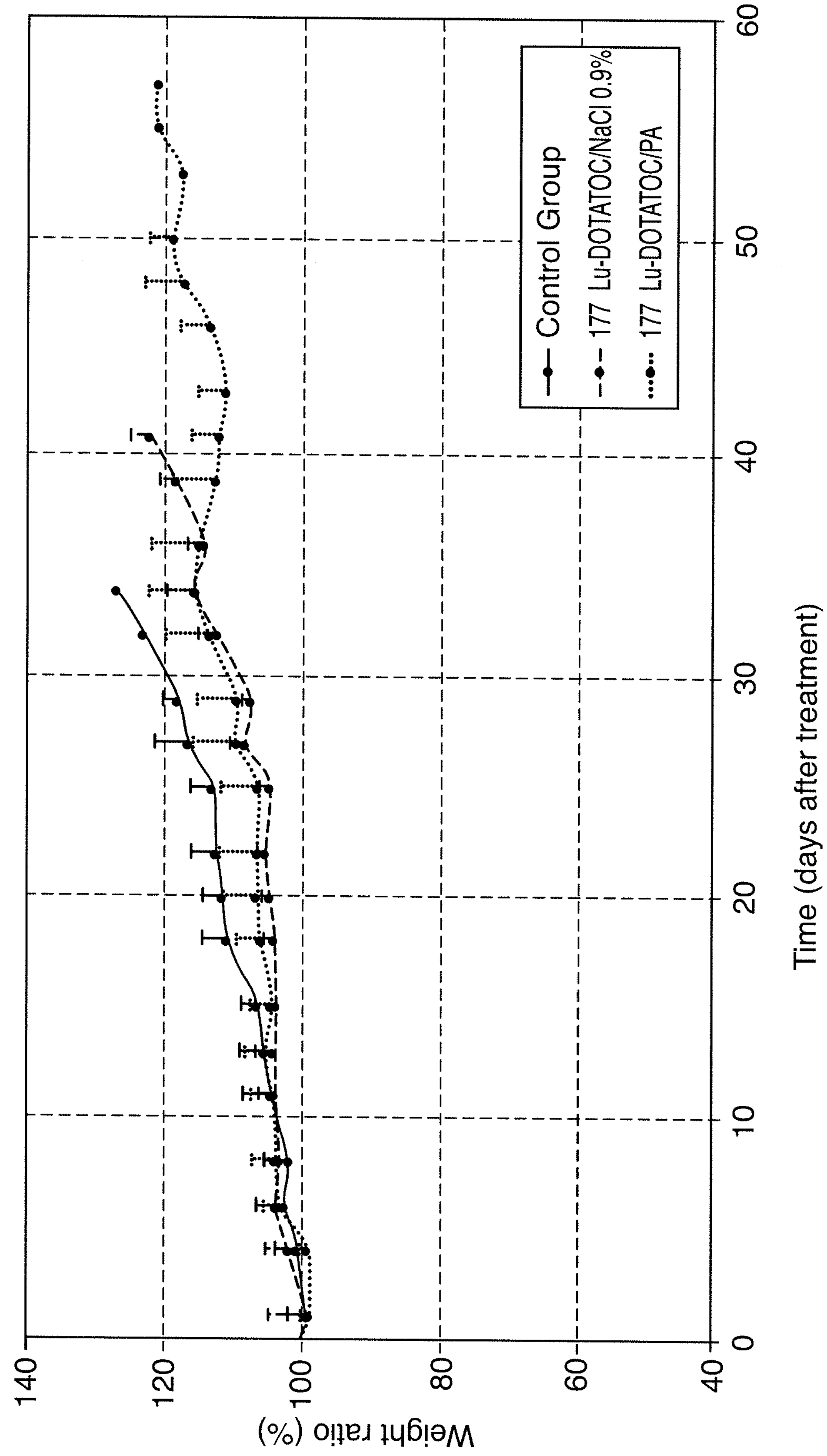

Control Group
177 Lu-DOTATOC/NaCl 0.9%
177 Lu-DOTATOC/PA
Weight ratio (%)
140
120
100
80
60
40
Time (days after treatment)
0
10
20
30
40
50
60


Figure 3

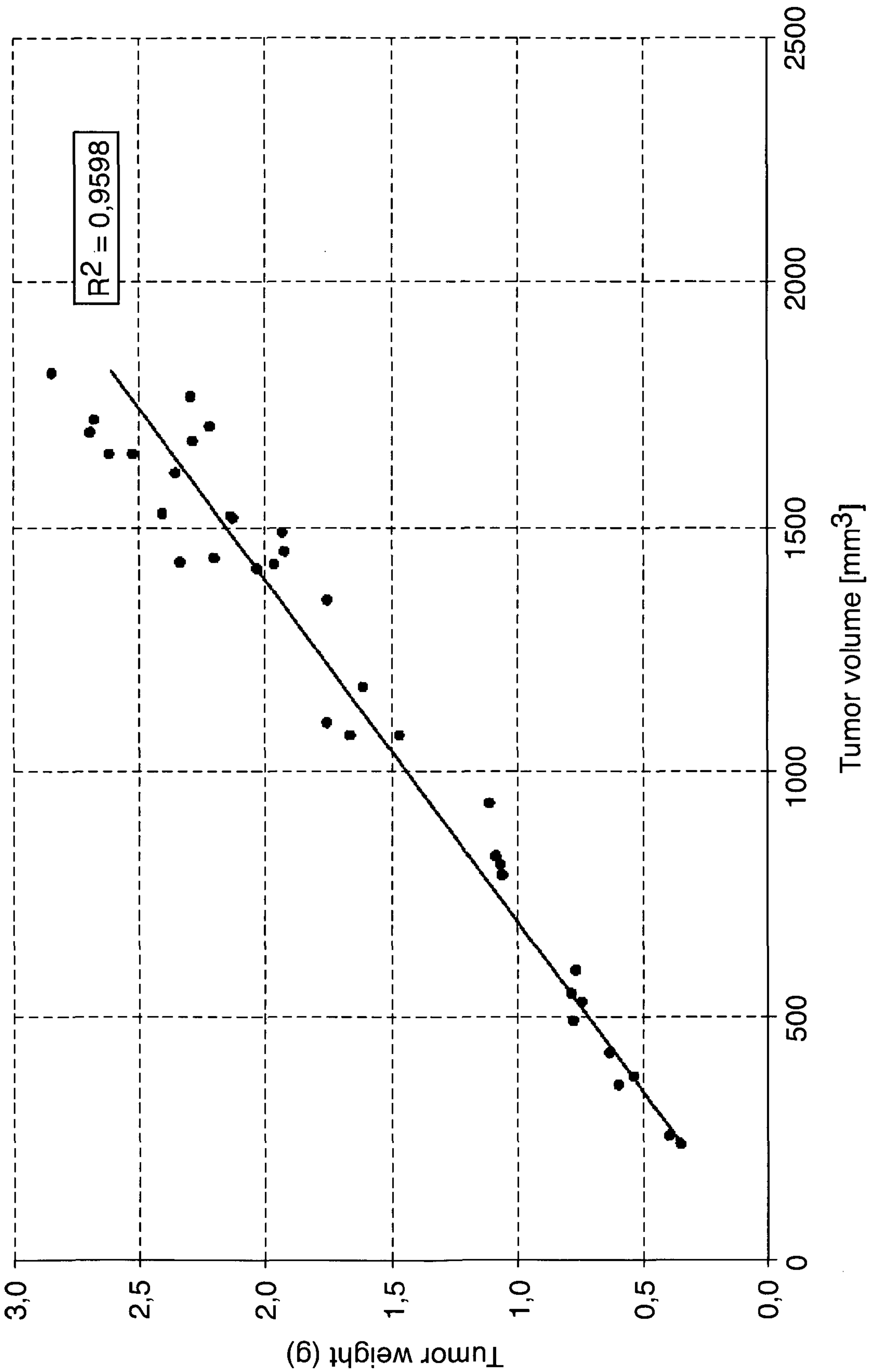

$R^2 = 0,9598$
Tumor weight (g)
0,0
0,5
1,0
1,5
2,0
2,5
3,0
Tumor volume [mm$^3$]
0
500
1000
1500
2000
2500


Figure 4

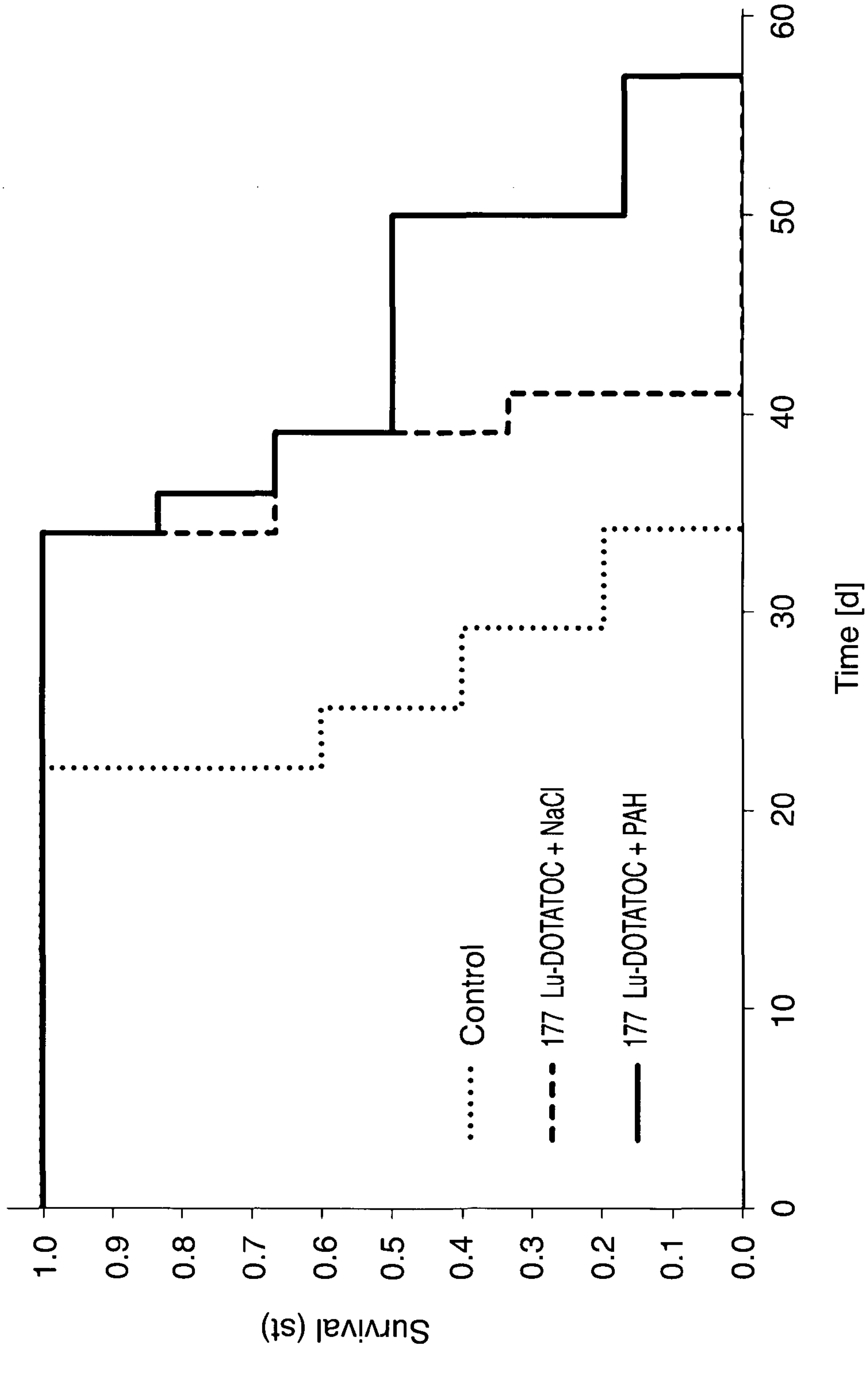
Control
177 Lu-DOTATOC + NaCl
177 Lu-DOTATOC + PAH
Survival (st)
Time [d]
1.0
0.9
0.8
0.7
0.6
0.5
0.4
0.3
0.2
0.1
0.0
0
10
20
30
40
50
60

Figure 5

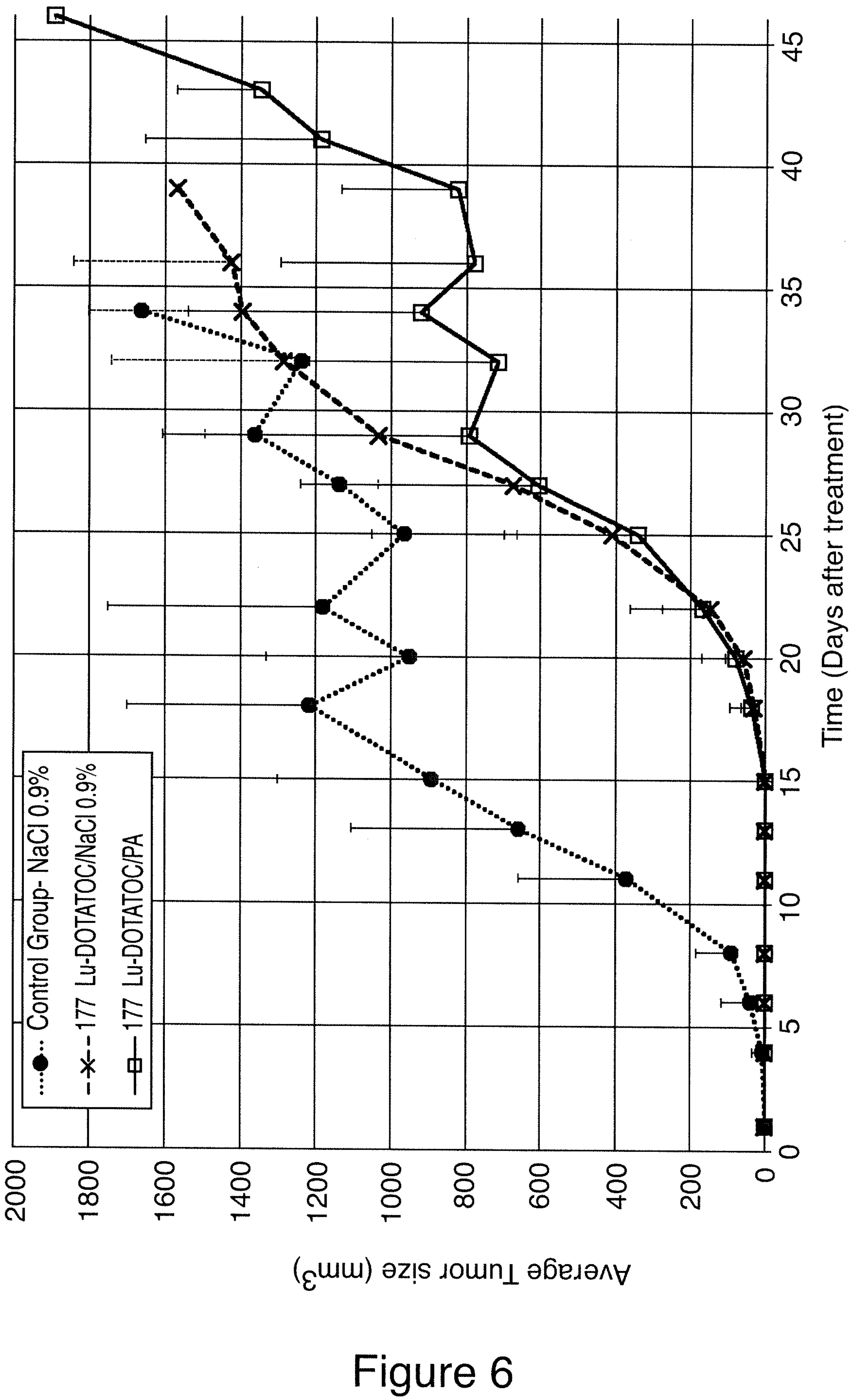
Control Group- NaCl 0.9%
177 Lu-DOTATOC/NaCl 0.9%
177 Lu-DOTATOC/PA
Average Tumor size (mm3)
2000
1800
1600
1400
1200
1000
800
600
400
200
0
0
5
10
15
20
25
30
35
40
45
Time (Days after treatment)

Figure 6

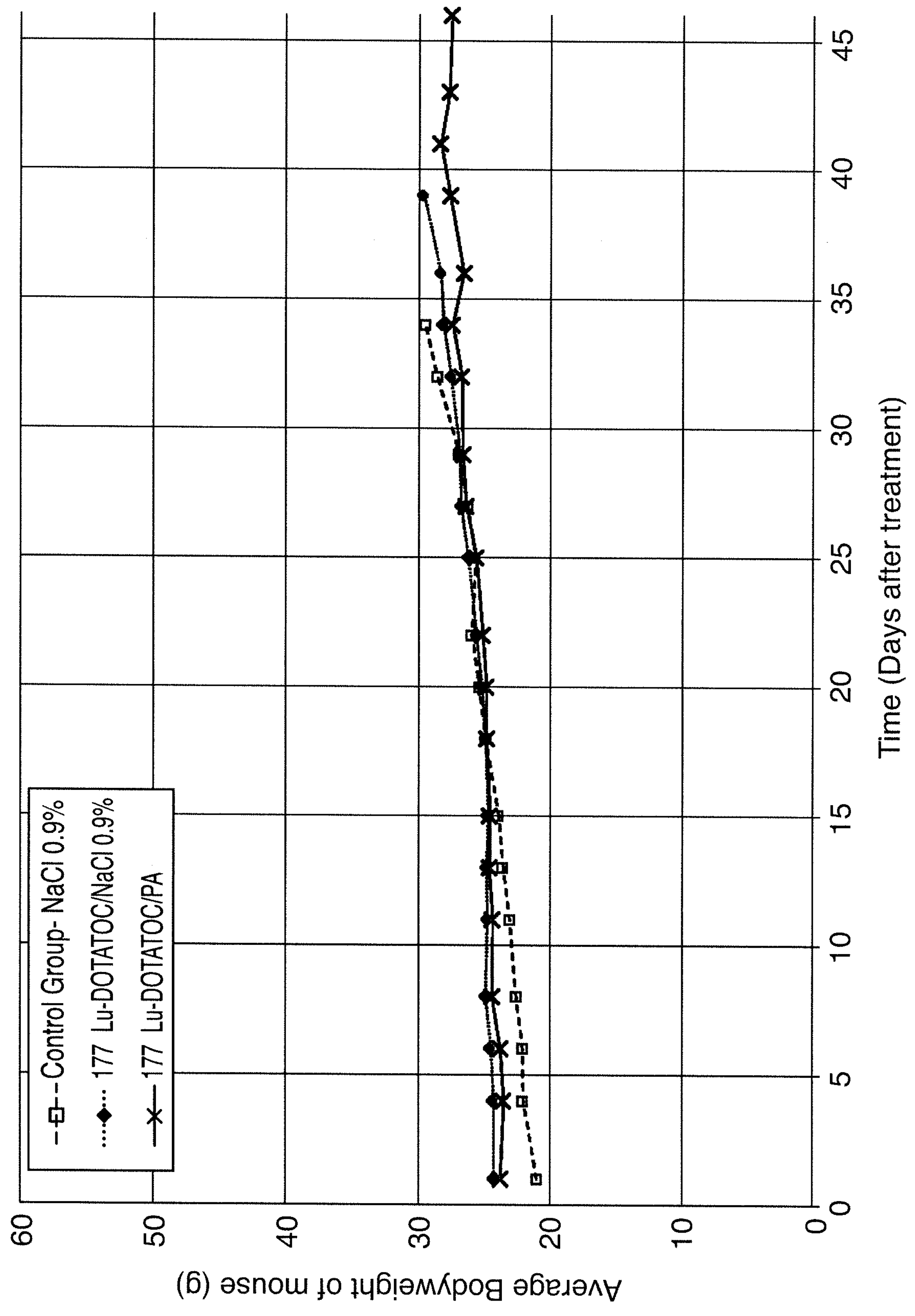

Control Group- NaCl 0.9%
177 Lu-DOTATOC/NaCl 0.9%
177 Lu-DOTATOC/PA
Average Bodyweight of mouse (g)
Time (Days after treatment)
0
10
20
30
40
50
60
0
5
10
15
20
25
30
35
40
45


Figure 7

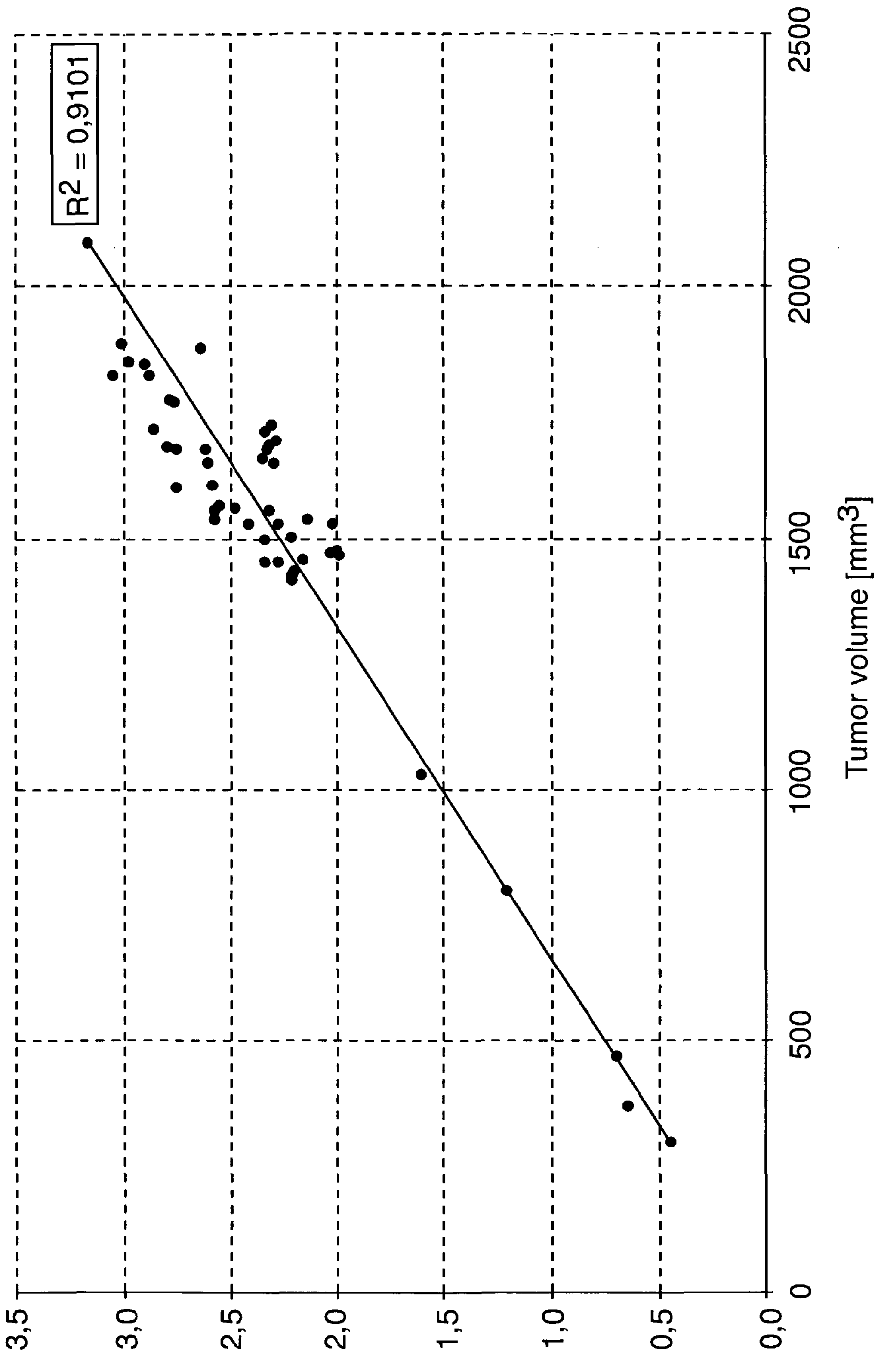

$R^2 = 0,9101$
Tumor weight (g)
3,5
3,0
2,5
2,0
1,5
1,0
0,5
0,0
Tumor volume [mm3]
0
500
1000
1500
2000
2500


Figure 8

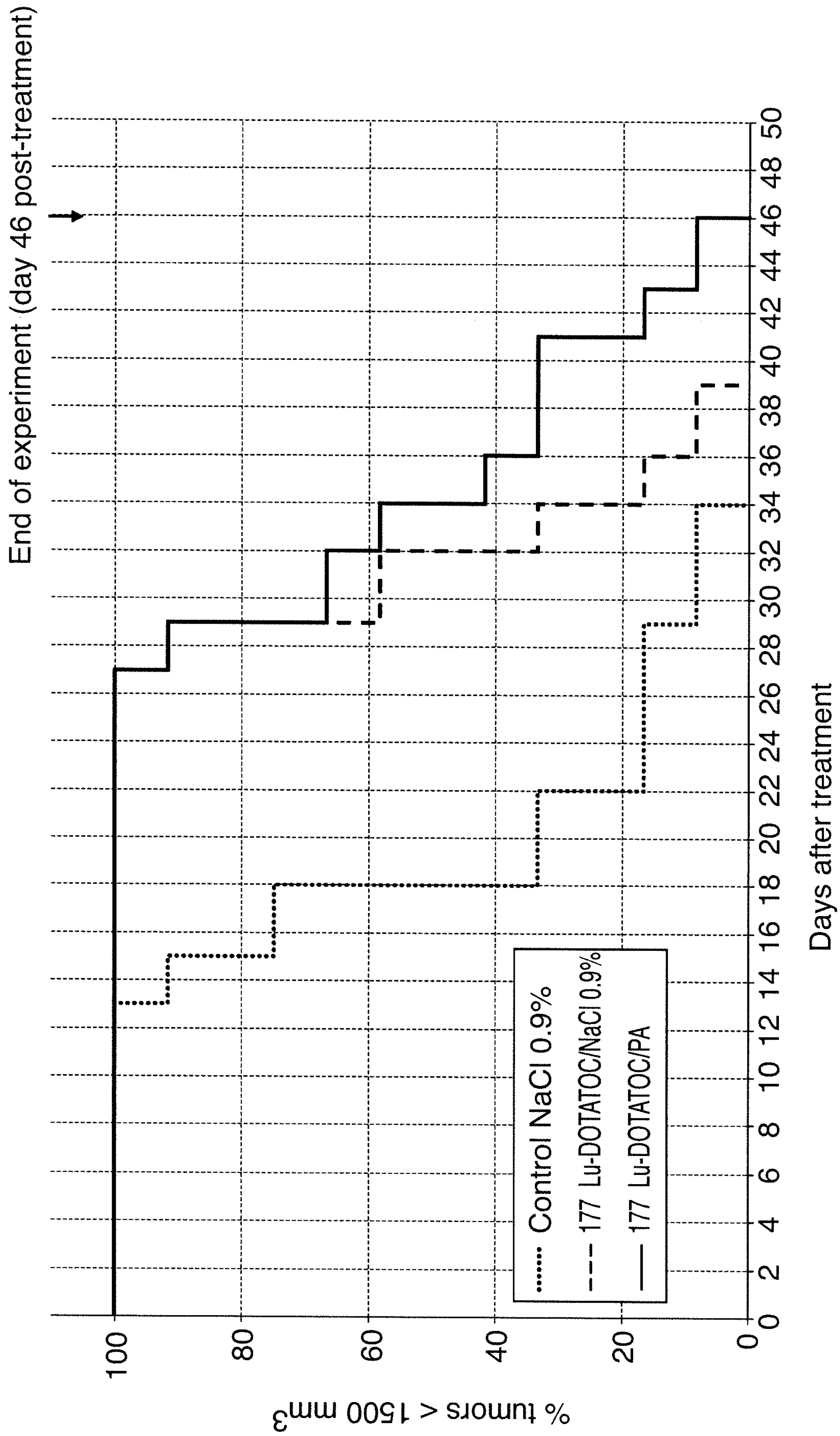
End of experiment (day 46 post-treatment)
% tumors < 1500 mm3
0
20
40
60
80
100
Days after treatment
0 2 4 6 8 10 12 14 16 18 20 22 24 26 28 30 32 34 36 38 40 42 44 46 48 50
Control NaCl 0.9%
177 Lu-DOTATOC/NaCl 0.9%
177 Lu-DOTATOC/PA

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2020062950 W
- WO 2018215627 A1
- WO 2013022797 A1
- WO 2015055318 A1
- EP 2862857 A1
- EP 2970345 A1
- WO 2020109523 A1


### Non-patent literature cited in the description


- **BAUM et al.** *Theranostics,* 2016, vol. 6 (4), 501
- **NATOCHIN et al.** *Comp. Biochem. Physiol,* 1989, vol. 94C (1), 115-120
- **KERDJOUDJ et al.** *Dalton Trans.,* 2016, vol. 45, 1398-1409
- **CHANG.** *Rev Urol.,* 2004, vol. 6 (10), S13-S18
- **BOUCHELOUCHE et al.** *Discov Med.,* January 2010, vol. 9 (44), 55-61
- **HILLIER et al.** *Cancer Res.,* 01 September 2009, vol. 69 (17), 6932-40
- **BENEŠOVÁ et al.** *JNM,* 2015, vol. 56, 914-920
- **BARRETT JA et al.** *J Nucl Med.,* 2013, vol. 54, 380-387
- **ZECHMANN et al.** *Eur J Nucl Med Mol Imaging,* 2014, vol. 41, 1280-1292
- **AFSHAR-OROMIEH A et al.** *J Nucl Med.,* 2015, vol. 56, 1697-1705
- **WEINEISEN M et al.** *J Nucl Med.,* 2015, vol. 56, 1169-1176
- **ROBU 5 et al.** *J Nucl Med.,* 2017, vol. 58, 235-242
- **CHEN Y et al.** *Clin Cancer Res.,* 2011, vol. 17, 7645-7653
- **GIESEL FL et al.** *Eur J Nucl Med Molecular Imaging,* 2017, vol. 44, 678-688
- **KELLY et al.** Dual-Target Binding Ligands with Modulated Pharmacokinetics for Endoradiotherapy of Prostate Cancer. *J Nucl Med.,* September 2017, vol. 58 (9), 1442-1449
- **CHOY et al.** *Theranostics,* 2017, vol. 7 (7), 1928-1939
- **CAPELLO A et al.** Tyr3-octreotide and Tyr3-octreotate radiolabeled with Lu or Y: peptide receptor radionuclide therapy results in vitro. *Cancer Biother Radiopharm,* October 2003, vol. 18 (5), 761-8
- **GUO et al.** *J Nucl Med.,* 1999, vol. 40, 1563-1569
- **MATHIAS et al.** *Bioconjug Chem.,* 2000, vol. 11, 253-257
- **LEAMON et al.** *Bioconjug Chem.,* 2002, vol. 13, 1200-1210
- **REDDY et al.** *J Nucl. Med.,* 2004, vol. 45, 857-866
- **MÜLLER et al.** *J Nucl Med Mol Imaging,* 2006, vol. 33, 1007-1016
- **MÜLLER et al.** *Bioconjug Chem.,* 2006, vol. 17, 797-806
- **SIEGEL et al.** *J Nucl Med.,* 2003, vol. 44, 700-707
- **MATHIAS et al.** *Nucl Med Biol.,* 1999, vol. 26, 23-25
- **MATHIAS et al.** *Nucl Med Biol.,* 2003, vol. 30, 725-731
- **BETTIO et al.** *J Nucl Med.,* 2006, vol. 47, 1153-1160
- **BAUM RP et al.** *The Journal of Nuclear Medicine,* May 2018, vol. 59 (5
- **ZHANG H et al.** *Cancer Res,* 2004, vol. 64, 6707-6715
- **BODEI L et al.** *Eur J Nucl Med Mol Imaging,* 2007, vol. 34 (2), S221
- **HENNIG IM et al.** *Int J Cancer,* 1995, vol. 61, 786-792
- **KNEIFEL S.** *Eur J Nucl Med Mol Imaging.,* 2007, vol. 34, 1388-1395
- **CORDIER et al.** *Eur J Nucl Med Mol Imaging.,* 2010, vol. 37, 1335-1344